# EUROPEAN PATENT SPECIFICATION

(11) **EP 3 094 688 B1**
(45) Date of publication and mention of the grant of the patent: **13.09.2017**
(21) Application number: 15701256.8
(22) Date of filing: 13.01.2015
(51) Int. Cl.: C09B 57/00, C09K 11/06, H01L 51/00, H05B 33/14

(54) **HOLE TRANSPORTING AND LIGHT ABSORBING MATERIAL FOR SOLID STATE SOLAR CELLS**
LOCHTRANSPORTIERENDES UND LICHTABSORBIERENDES MATERIAL FÜR HALBLEITERSOLARZELLEN
MATÉRIAU DE TRANSPORT DE TROUS ET D'ABSORPTION DE LUMIÈRE POUR CELLULES SOLAIRES À L'ÉTAT SOLIDE

(30) Priority: 16.01.2014 EP 14151392
(43) Date of publication of application: 23.11.2016
(73) Proprietor: Ecole Polytechnique Fédérale de Lausanne (EPFL), 1015 Lausanne (CH)
(72) Inventor: QIN, Peng, 122500 Lingyuan, Liaoning Province (CN); NAZEERUDDIN, Mohammad Khaja, CH-1024 Ecublens (CH); GRAETZEL, Michael, CH-1025 St-Sulpice (CH); KO, Jaejung, Cheongju-si, Seoul Gyeongsangbuk-do 361-858 (KR); PAEK, Sanghyun, Hwaseong-si, Seoul Gyeonggi-do 445-170 (KR); CHO, Nara, Seoul, 138-891 (KR)
(74) Representative: Ganguillet, Cyril
(86) International application number: PCT/IB2015/050245
(87) International publication number: WO 2015/107454

(56) References cited:
- EP-A1- 1 990 373
- EP-A2- 2 182 040
- EP-A2- 2 248 869
- WO-A2-2009/098643
- WO-A2-2011/107186
- DE-A1- 19 808 088
- KR-B1- 101 172 374

## Description

### Technical Field

The present invention relates to hole transporting and light absorbing material, to hole transporting and light absorbing material for solid state photovoltaic devices, in particular solid state solar cells, and for thin-film photovoltaic devices and organic-inorganic perovskite films or layer photovoltaic devices, to a solid-state heterojunction and flat junction, to a solid state solar cell and to a method for preparing said solid state solar cell.

### Prior Art and the Problem Underlying the Invention

The conversion of solar energy to electrical current using thin film third generation photovoltaics (PV) is being widely explored for the last two decades. The sandwich/monolithic-type PV devices, consisting of a mesoporous photoanode with an organic/inorganic light harvester, redox electrolyte/solid-state hole conductor, and counter electrode, have gained significant interest due to the ease of fabrication, flexibility in the selection of materials and cost effective production. Recently, the organometallic halide perovskite based on tin (CsSnX₃) or lead (CH₃NH₃PbX₃) (Etgar, L. et al.; J. Am. Chem. Soc. 2012, 134, 17396-17399), have been introduced as light harvester to replace traditional metal-organic complex or organic molecules. The lead perovskite shows a power conversion efficiency (PCE) of 6.54% in liquid electrolyte based devices, while 12.3% in solid state devices (Noh, J. H. et al.; Nano Lett. 2013). Unpublished European patent application EP 12179323.6 disclosed a solid-state solar cell comprising one or more organic-inorganic perovskite layers and showing remarkable conversion efficiencies even though in absence of organic hole transporting material.

In these solid state photovoltaic devices, the perovskite pigment is usually applied from a solution of two precursors of the perovskite pigment, PbX₂ (X = I, Br or Cl) and CH₃NH₃I, in a common solvent. The optimal protocol for the deposition of CH₃NH₃PbX₃ on TiO₂ is achieved by the spin-coating of the precursor (CH₃NH₃X and PbX₂, X = Cl, Br, I) solution on the mesoporous TiO₂ film, followed by low temperature annealing step which results in a crystalline CH₃NH₃PbX₃ (Noh et al.). From experience, the morphology of the perovskite crystals formed during this kind of solution processing cannot be well controlled and is one of the reasons for the poor reproducibility of photovoltaic cell performance. Unpublished European patent application EP 13166720.6 disclosed an efficient and reproducible method for the application of the light harvester layer of perovskite on the nanopourous layer of the current collector. The two precursors of the organic-inorganic perovskite being in solution are separately applied on the nanoporous layer of the current collector in a two-step deposition, namely a first step for forming a film on the nanoporous layer with the first precursor and a second step for applying a film of the second precursor, to obtain a layer comprising the organic-inorganic perovskite pigment. Recently, solid-state solar cells being prepared according to this method and comprising the hybrid organic-inorganic perovskite CH₃NH₃PbX₃, X being Cl⁻, Br⁻ or I⁻, in combination with spiro-MeOTAD (2,2',7,7'-tetrakis(N,N-di-p-methoxyphenylamine) -9,9'-spirobifluorene) as organic hole transporting material (HTM) achieve power conversion efficiency (PCE) of 15% under full illumination.

However the use of spiro-MeOTAD as hole transporting material may trigger instability in such solid-state solar cells. Because Spiro-MeOTAD has two oxidation potentials being very close, this hole transporting material in the oxidized form is able to forms a dication, which in turn can dismutate and might cause device instability. The use of other HTMs such as polytriarylamine (PTAA) and poly(3-hyxalthiophen) (P3HT) in combination with perovskite pigment has resulted in photovoltaic device showing a PCE up to 12%.

US 2009/0295275 discloses compounds based on quinozilino acridine, which are used as hole transporter material in organic electroluminescent devices and which, for most of them are colorless or emitting in the blue.

EP 2248869A2, EP 2182040A2 and WO 2011/107186 A2 disclose compounds based on quinozilino acridine for OLED devices, wherein the donor moiety is not separated from the acceptor moiety and the acceptor moiety is not further substituted.

The present invention addresses the disadvantage of organic hole transporting material, which provides instability to the device, when said hole transporter material is in oxidized form, as it is the case of spiro-MeOTAD.

The present invention also pursues to provide new hole transporting material, which provides a higher PCE to the solid-state photovoltaic devices comprising perovskite as sensitizer or light absorbing material.

The present invention addresses the disadvantage of the perovskite pigment, which cannot absorb the complete incident light, in particular in the visible part of the light spectrum and to absorb the remnant light passing through the layer comprising the perovskite pigment to increase the photoconversion and photocurrent generation of the whole device and therefore the efficiency and the performance of the photovoltaic device.

The invention pursues to provide an efficient solar cell, which can be rapidly prepared in an efficient way, using readily available or low cost materials such as conductive material, using a short manufacturing procedure based on industrially known manufacturing step, keeping the material costs and the material impact on the environment very low.

The present invention addresses the problems depicted above.

### Summary of the Invention

Remarkably, in some aspects, the present inventors have found that a compound based on quinolizino acridine operates as a hole transporting material and as a light harvester or a light absorbing material and improves the PCE of solid photovoltaic devices comprising perovskite pigment as sensitizer. Said compound may absorb light to the near IR regions of the light spectrum, namely from 400 nm to 920 nm. In a solid state photovoltaic device, said compound also absorbs the remanent light passing through a sensitizer layer and in particular a particular a sensitizer layer comprising perovskite pigment.

The specific configuration of the structure of the compound based on quinolizino acridine being the donor moiety separated from the acceptor moieties provides a fine tuning of the free charges extracted from the sensitizer layer, in particular from the perovskite layer. Although their large size, said compounds are good soluble in organic solvent, which greatly facilitates their purification and processing and their application or deposition on the sensitizer layer in the solid photovoltaic device.

In an aspect, the present invention provides compound of formula (I): 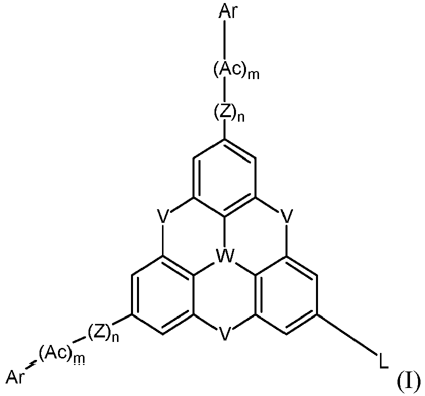 wherein
- W is a nitrogen (N) atom;
- V is, on each occurrence, identically or differently selected from C(R¹)₂, C=O or O, R¹ being selected from C1-C12 alkyl;
- L is selected from moiety of formula ------(Z)ₙ-(Ac)ₘ-Ar as defined below or from moiety of formula (19) 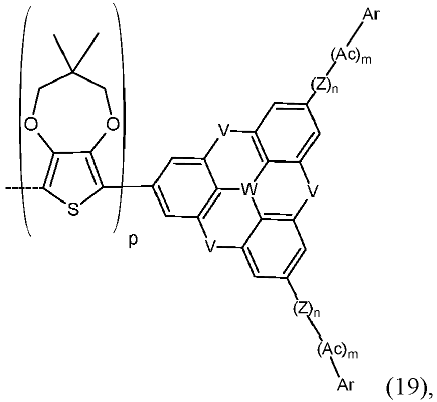 p being 0, 1 or 2 and moiety of formula ------(Z)ₙ-(Ac)ₘ-Ar being as defined below;
- Z is a heteroaromatic ring system, on each occurrence, identically or differently selected from C4-C20 heteroaryl, C4-C20 heteroaryloxy group, wherein the heteroatoms are selected from O, S, Se, Si and wherein said heteroaryl, aryloxy group, heteroaryloxy group are substituted by C1-C20 alkyl, C1-C20 heteroalkyl, C2-C20 alkenyl or C2-C20 alkynyl, wherein said alkyl, alkenyl, and alkynyl, if they comprise 3 or more carbons, may be linear, branched or cyclic, and n is 0, 1, 2, or 3;
- Ac is an acceptor group, on each occurrence, identically or differently selected from a moiety according to any one of the formulae (1) to (18), 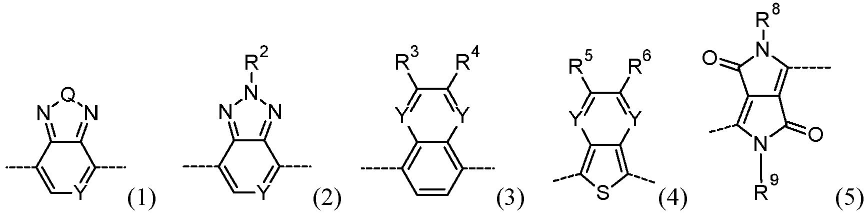 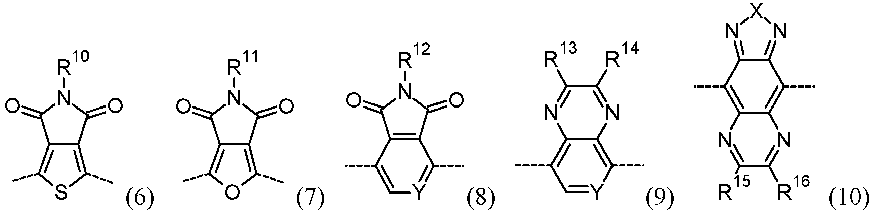 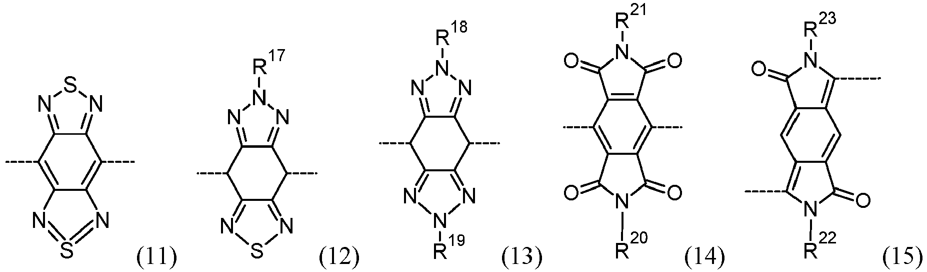 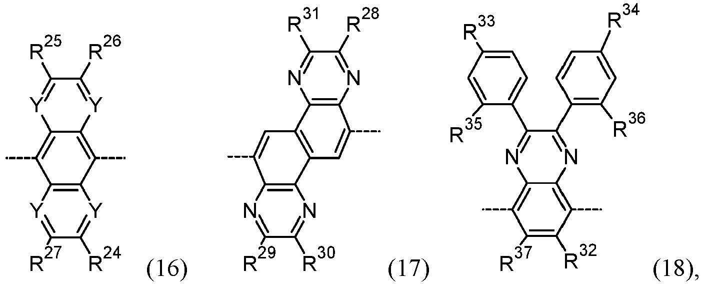
- wherein
   - R² to R³⁷ are substituents independently selected from H, ether group, C1-C16 alkyl, C1-C16 alkoxy group, C1-C16 thioalkyl, C1-C16 alkoxyalkyl, C4-C16 aryl, C1-C16 arylalkyl or C4-C16 heteroaryl, C4-C16 heteroarylalkyl, the heteroatoms being selected from O, S, or N,
   - Q is selected from S, O, Se, Si or N and when Q is N, said nitrogen atom is substituted by substituent as defined for R² to R³²,
   - Y is, on each occurrence, identically or differently selected from N or C, being unsubstituted or substituted by halogen selected from F, Cl, I, and Br, and
   - m is 0 or 1;
- -Ar is is a successive bound heteroaromatic rings system comprising from 2 to 10 heteroaromatic rings, said heteroaromatic rings being, on each occurrence, identically selected from C4-C20 heteroaryl and C4-C20 heteroaryloxy group, wherein the heteroatoms are selected from O, S, Se, Si and wherein said heteroaryl and heteroaryloxy groups are unsubstituted or substituted by -COOH, C4-C16 cyanoalkenyl carboxylic acid, C1-C20 alkyl, C1-C20 heteroalkyl, C2-C20 alkenyl or C2-C20 alkynyl, wherein said alkyl, alkenyl, and alkynyl, if they comprise 3 or more carbons, may be linear, branched or cyclic. According to an embodiment, the invention provides more specifically a compound of formula (II):

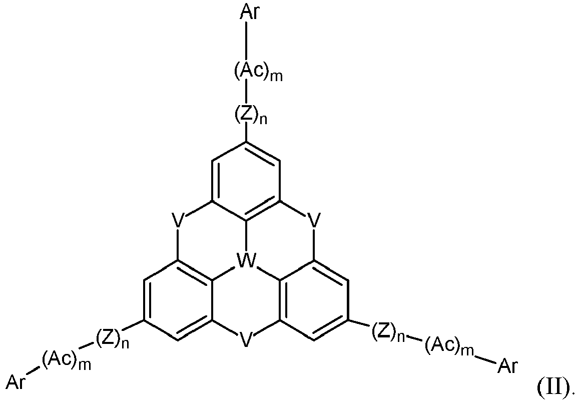

In an aspect, the invention provides a photovoltaic solid state device comprising a compound of the invention of formula (I), or in particular of formula (II).

In an aspect, the invention specifically provides a photovoltaic solid state device comprising a compound of the invention of formula (I), or in particular of formula (II) and further comprising an organic-inorganic perovskite as sensitizer, said perovskite being under the form of layer.

In another aspect, the invention provides use of a compound of the invention as a hole transporting and light absorbing material in photovoltaic solid state device.

Further aspects and preferred embodiments of the invention are detailed herein below and in the appended claims. Further features and advantages of the invention will become apparent to the skilled person from the description of the preferred embodiments given below.

### Brief Description of the Drawings

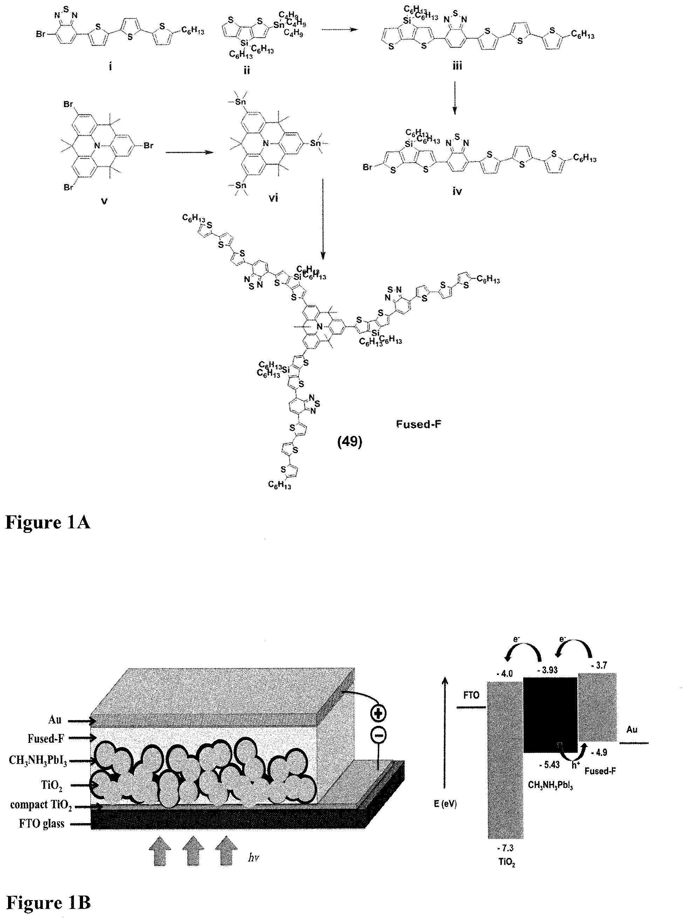

**Figure 1A** shows schematic diagram for the synthesis of compound of formula (49). **Figure 1B** shows, on the left, the schematic architecture of a photovoltaic device of the invention, in particular a solid solar cell comprising the following layers from the side of the light exposure: FTO glass being coated by a compact TiO₂ layer, on which mesoporous TiO₂ is provided with the sensitizer, an organic-inorganic perovskite CH₃NH₃PbI₃, on which the HTM (hole transporting material) being compound of formula (49), also named Fused-F, is spin-coated, and a metal layer being Au is provided on HTM, and, on the right, said Figure 1B shows the corresponding energy level diagram of the photovoltaic device.

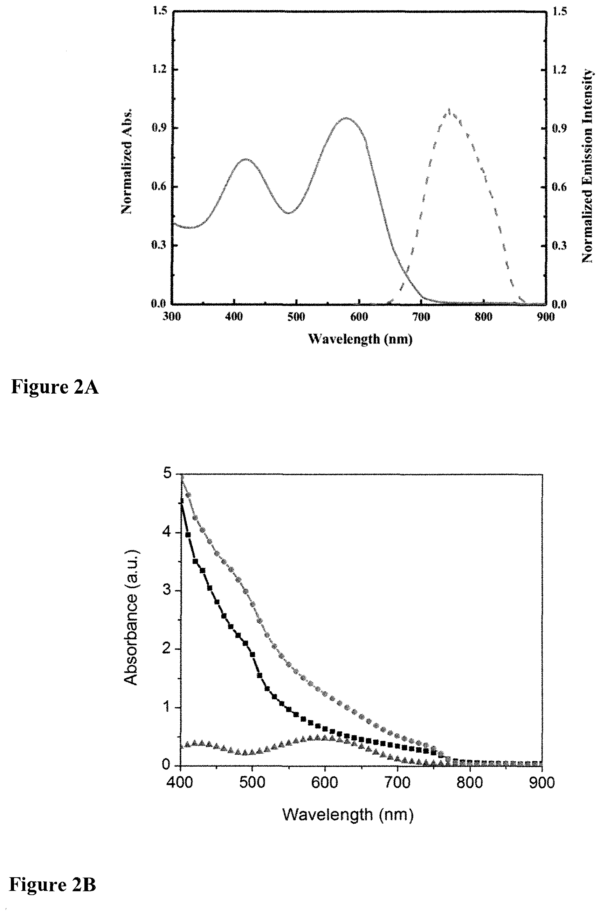

**Figure 2A** shows UV-Visible absorption (continuous line) and fluorescence (dashed line) spectra of of compound of formula (49)(Fused-F) in chloroform. Figure 2B shows UV-Visible absorption spectra of Fused-F (compound of formula (49)) coated on mesoporous TiO₂ (line with triangles) and TiO₂/CH₃NH₃PbI₃ (line with round dots) films. TiO₂/CH₃NH₃PbI₃ (line with squares) film without HTM is shown for comparison.

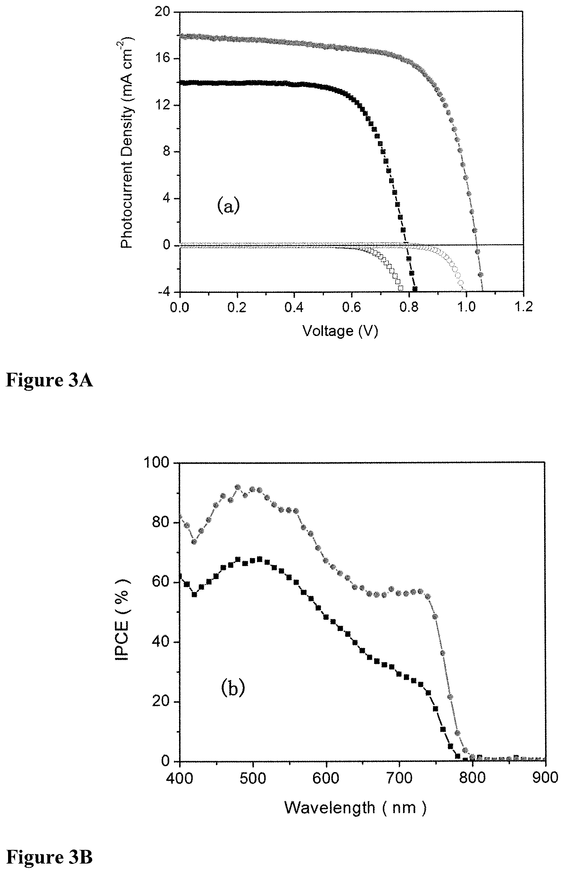

**Figure 3A** shows the Current-Voltage characteristics of the photovoltaic device with (round) and without (square) Fused-F(compound of formula (49)) measured in the dark (unfilled) and under 100 mW.cm⁻² photon flux (AM 1.5G) (filled). Figure 3B shows the incident photo-to-electron conversion efficiency spectra of the photovoltaic device with (round) and without (square) Fused-F (compound of formula (49)).

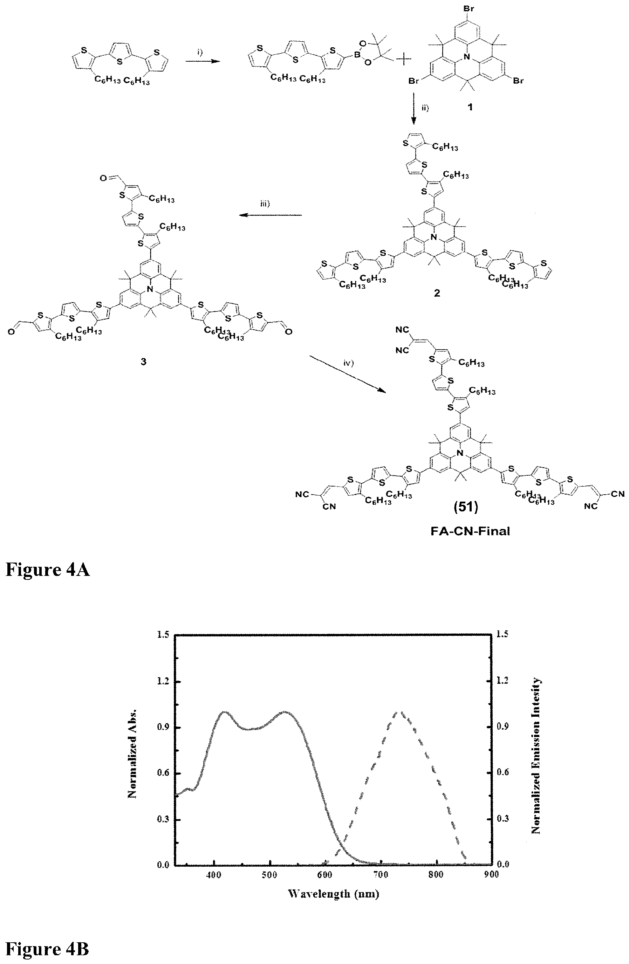

**Figure 4A** shows schematic diagram for the synthesis of preference compound of formula (51), also named FA-CN-Final. **Figure 4B** shows UV-Visible absorption (continuous line) and fluorescence (dashed line) spectra of reference compound of formula (51)(FA-CN-Final) in chloroform.

### Detailed Description of the Preferred Embodiments

The present invention concerns a compound based on quinolizino acridine of formula (I). 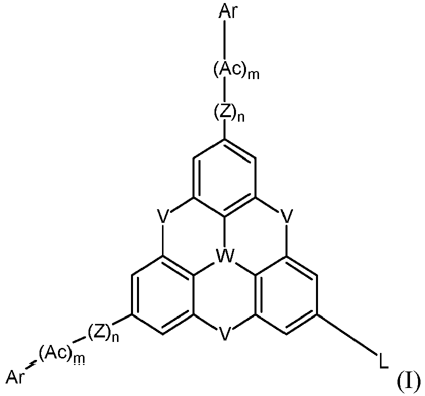 wherein
- W is a nitrogen (N) atom;
- V is, on each occurrence, identically or differently selected from C(R¹)₂, C=O or O, R¹ being selected from C1-C12 alkyl and C1 alkyl;
- m is an integer selected from 0 or 1; and
- n is an integer selected from 0, 1, 2, or 3.

L is a linked moiety selected from moiety of formula ------(Z)ₙ-(Ac)ₘ-Ar as defined below or from moiety of formula (19) 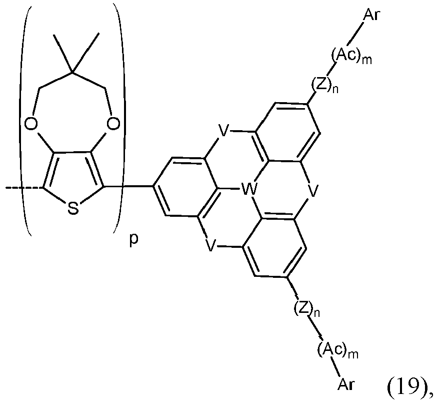 wherein p is an integer selected from 0, 1 or 2.

The compound of formula (I) may be a monomer, when L is a moiety of formula ------(Z)ₙ-(Ac)ₘ-Ar, or a dimer, when L is a moiety of formula (19). Said monomer is represented by a compound of formula (II)

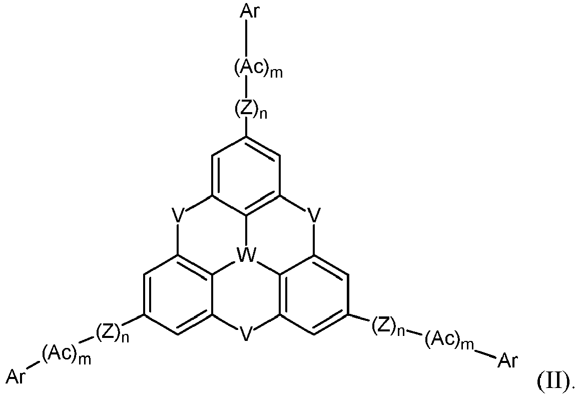

In the compound of the invention of formula (I) and/or of formula (II), W is preferably a nitrogen (N) atom.

In the compound of the invention of formula (I) and/or of formula (II), V is at least preferably, on each occurrence, identically selected from C(R¹)₂, C=O or O, R¹ being selected from C1-C12 alkyl and C1 alkyl. V is identical on each occurrence and represents selected from C(R¹)₂, R¹ being selected from C1-C12 alkyl, C4-C12 alkyl, C6-C10 alkyl and C1 alkyl, preferably C1 alkyl.

In the compound of formula (I), when L is selected from moiety of formula ------(Z)ₙ-(Ac)ₘ-Ar, said moiety is identical to one or two of the two other moieties ------(Z)ₙ-(Ac)ₘ-Ar substituted the quinolizino acridine core of the compound of formula (I). In the compound of formula (I) and/or in the compound of formula (II), said moieties of formula ------(Z)ₙ-(Ac)ₘ-Ar may be identical or different on each occurrence.

In the compound of formula (I), when L is selected from moiety of formula (19), p may be 0, 1, or 2, preferably 0 and the moiety of formula ------(Z)ₙ-(Ac)ₘ-Ar may be identical or different on each occurrence on the quinolizino acridine core of moiety of formula (19) and/or on the quinolizino acridine core of moiety of formula (I).

Ac is an acceptor group, on each occurrence, identically or differently selected from a moiety according to any one of the formulae (1) to (18), 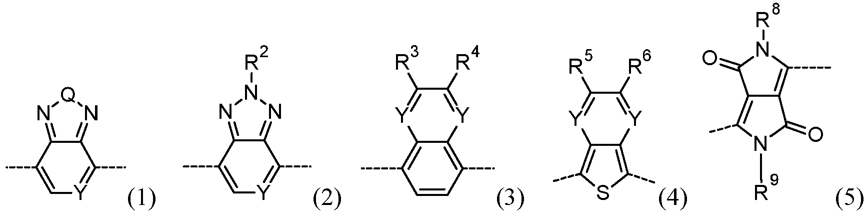 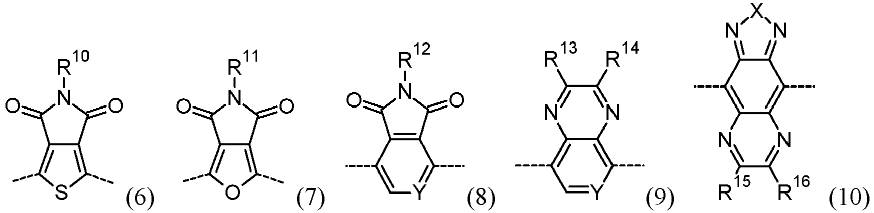 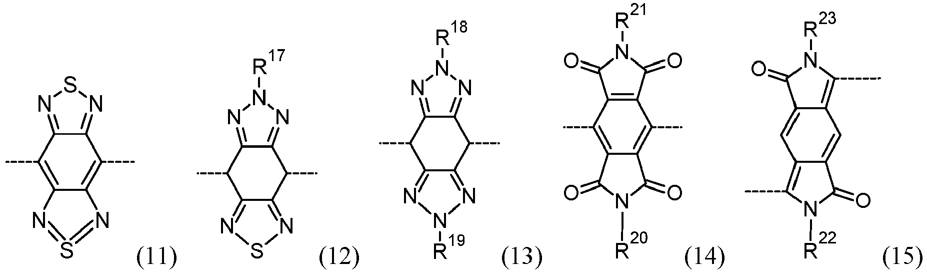 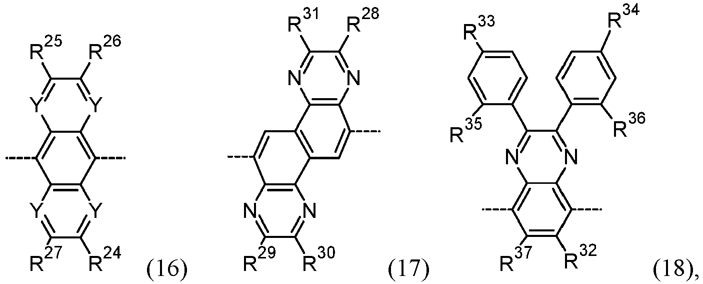
- wherein
   - R² to R³⁷ are substituents independently selected from H, ether group, C1-C16 alkyl, C1-C16 alkoxy group, C1-C16 thioalkyl, C1-C16 alkoxyalkyl, C4-C16 aryl, C1-C16 arylalkyl or C4-C16 heteroaryl, C4-C16 heteroarylalkyl, the heteroatoms being selected from O, S, or N,
   - Q is selected from S, O, Se, Si or N and when Q is N, said nitrogen atom is substituted by substituent as defined for R² to R³⁷,
   - Y is, on each occurrence, identically or differently selected from N or C, being unsubstituted or substituted by halogen selected from F, Cl, I, and Br, preferably F or Cl.

Preferably alkyl, alkoxy, thioalkyl, alkoxyalkyl, arylalkyl and heteroaryl of substituents R²-R³⁷ are selected from hydrocarbon containing from 1 to 16 carbons, 1 to 12 carbons or 1 to 8 carbons. Substituents from R²-R³⁷ substituting the same moiety of same formula may be identical to the other substituent substituting the same moiety of same formula or different. For example, R³ and R⁴ substituting the moiety of formula (3) may be identical or different.

According to a further embodiment, the acceptor group Ac is selected from a moiety according to any one of the formulae (1) to (4) and (10) to (13) as defined above. Ac is preferably selected from a moiety according to any one of the formulae (1), (10) and (11) as defined above or most preferably is moiety of formula (1).

According to an embodiment, the acceptor group Ac is selected from a moiety according to any one of the formulae (1) to (18) as defined above, wherein Q is selected from S, O, Se, Si, or from S, O, or Se.

According to further embodiment, the acceptor group Ac is selected from a moiety according to any one of the formulae (1) to (18) as defined above, wherein Y is C atom, preferably said C atom being further substituted by halogen.

In another embodiment, the acceptor group Ac is selected from a moiety according to any one of the moiety of formula (1), wherein Q is S and Y is C atom.

Z is a heteroaromatic ring system, on each occurrence, identically or differently selected from C4-C20 heteroaryl, C4-C20 aryloxy group, C4-C20 heteroaryloxy group, wherein the heteroatoms are selected from O, S, Se, Si and wherein said heteroaryl, aryloxy group, heteroaryloxy group are substituted by C1-C20 alkyl, C1-C20 heteroalkyl, C2-C20 alkenyl or C2-C20 alkynyl, wherein said alkyl, alkenyl, and alkynyl, if they comprise 3 or more carbons, may be linear, branched or cyclic.

Z is a heteroaromatic ring system, on each occurrence, identically or differently selected from C4-C20 heteroaryl and C4-C20 heteroaryloxy group, wherein the heteroatoms are selected from O, S, Se, Si and wherein said heteroaryl and heteroaryloxy group are substituted by C1-C20 alkyl, C1-C20 heteroalkyl, C2-C20 alkenyl or C2-C20 alkynyl, wherein said alkyl, alkenyl, and alkynyl, if they comprise 3 or more carbons, may be linear, branched or cyclic.
According to another embodiment, the heteroaromatic ring system Z of the compound of formula (I) and/or the compound of formula (II) is selected from a moiety according to any one of the formulae (20) to (27) 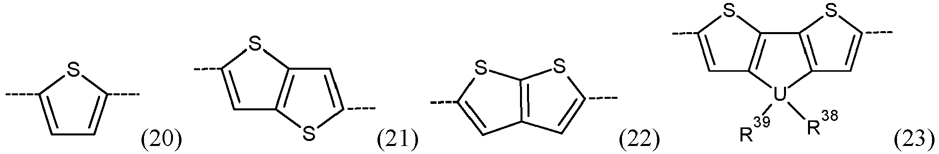 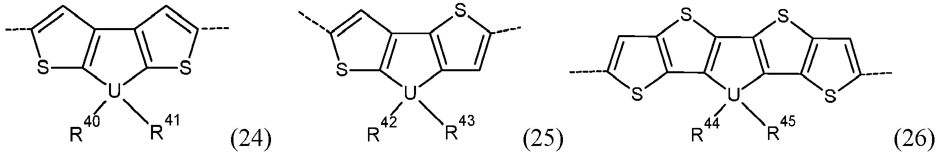 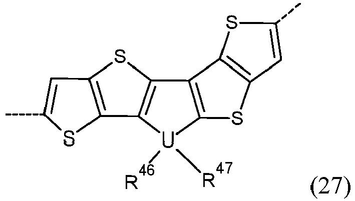
- wherein R³⁹ to R⁴⁷ are substituents independently selected from H, C1-C16 alkyl, C1-C16 alkoxy group, C1-C16 thioalkyl, C1-C16 alkoxyalkyl and if they comprise 3 or more carbons, they may be linear or branched and U is selected from S, Si or C.
In a further embodiment, Z is selected from a moiety according to any one of the formulae (20), (23) to (26).
In the compound of formula (I) and/or of formula (II), Ar is an aromatic ring system or a heteroaromatic ring system, on each occurrence, identically or differently selected from C4-C20 aryl, C4-C20 heteroaryl, C4-C20 aryloxy group, C4-C20 heteroaryloxy group, wherein the heteroatoms are selected from O, S, Se, Si and wherein said aryl, heteroaryl, aryloxy group, heteroaryloxy group are unsubstituted or substituted by -COOH, C4-C16 cyanoalkenyl carboxylic acid, C1-C20 alkyl, C1-C20 heteroalkyl, C2-C20 alkenyl or C2-C20 alkynyl, wherein said alkyl, alkenyl, and alkynyl, if they comprise 3 or more carbons, may be linear, branched or cyclic. In another embodiment, Ar is a heteroaromatic ring system, on each occurrence, identically or differently selected from C4-C20 heteroaryl, C4-C20 aryloxy group, C4-C20 heteroaryloxy group, wherein the heteroatoms are selected from O, S, Se or Si, preferably S and wherein said heteroaryl, aryloxy group, heteroaryloxy group are unsubstituted or substituted by -COOH, C4-C16 cyanoalkenyl carboxylic acid, C1-C20 alkyl, C1-C20 heteroalkyl, C2-C20 alkenyl or C2-C20 alkynyl, wherein said alkyl, alkenyl, and alkynyl, if they comprise 3 or more carbons, may be linear, branched or cyclic. Thus Ar as being a heteroaromatic ring system is or represents one heteroaromatic ring system (one unit) or a successive bound heteroaromatic rings system (several units) comprising from 2 to 10 heteroaromatic rings, preferably 3 heteroaromatic rings, said heteroaromatic rings being, on each occurrence, identically selected from C4-C20 heteroaryl and C4-C20 heteroaryloxy group, wherein the heteroatoms are selected from O, S, Se, Si and wherein said heteroaryl and heteroaryloxy groups are unsubstituted or substituted by -COOH, C4-C16 cyanoalkenyl carboxylic acid, C1-C20 alkyl, C1-C20 heteroalkyl, C2-C20 alkenyl or C2-C20 alkynyl, wherein said alkyl, alkenyl, and alkynyl, if they comprise 3 or more carbons, may be linear, branched or cyclic. For the meaning of the present application, Ar may represent one heteroaromatic ring system comprising one heteroaromatic ring or a successive bound heteroaromatic rings system comprising from 2 to 10 heteroaromatic rings, preferably 3 heteroaromatic rings, wherein the heteroaromatic rings or the last heteroaromatic rings of the successive bound heteroaromatic rings system may be further unsubstituted or substituted as defined above. For the meaning of the present application, the term "bound" in the successive bound heteroaromatic rings system does not mean "fused". Thus the successive heteroaromatic rings in the system are not fused but bound to each other.
The moiety Ar may represent one heteroaromatic ring (one unit) or successive bound heteroaromatic rings (several units), from 2 to 10 heteroaromatic ring, preferably 3 heteroaromatic rings, wherein said one heteroaromatic ring or the last heteroaromatic ring, if Ar is a successive bound heteroaromtic rings system, is substituted by -COOH, C4-C16 cyanoalkenyl carboxylic acid, C1-C20 alkyl, C1-C20 heteroalkyl, C2-C20 alkenyl or C2-C20 alkynyl, wherein said alkyl, alkenyl, and alkynyl, if they comprise 3 or more carbons, may be linear, branched or cyclic. Said last ring may be substituted by an anchoring group independently selected from -COOH, PO₃H₂, -PO₄H₂, -P(R⁴⁸)O₂H, - SO₃H₂, -SO₄H₂, -CONHOH⁻, 1,2-hydroxybenzene, 1-hydroxy-2-carboxybenzene, acetylacetonate, deprotonated forms of the aforementioned, organic and /or inorganic salts of said deprotonated forms, and chelating groups with π-conducting character. R⁴⁸ may be a hydrocarbon comprising from 1 to 50 carbons and 0-25 heteroatoms selected from O, N, or S, said hydrocarbon being covalently bound to the P atom of said phosphinic acid group by a carbon atom. R⁴⁸ may be substituted or unsubstituted, linear, branched or cyclic C1-C20 alkyl, C2-C20 alkenyl, C2-C20 alkynyl, and C4-C20 aryl.
According to a further embodiment, the compound of formula (I) and/or the formula (II) is selected from a compound according to any one of formulae (49) to (52), preferably a compound according to any one of the formulae (49) and (50) (the compound of formula (51) is a reference compound):

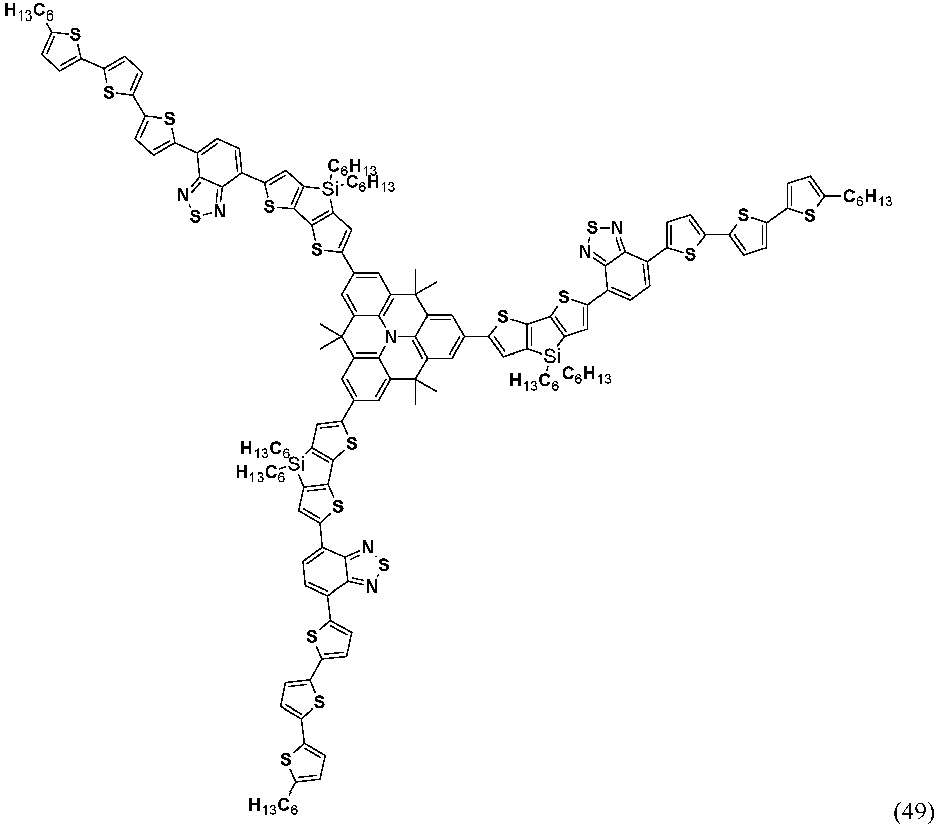

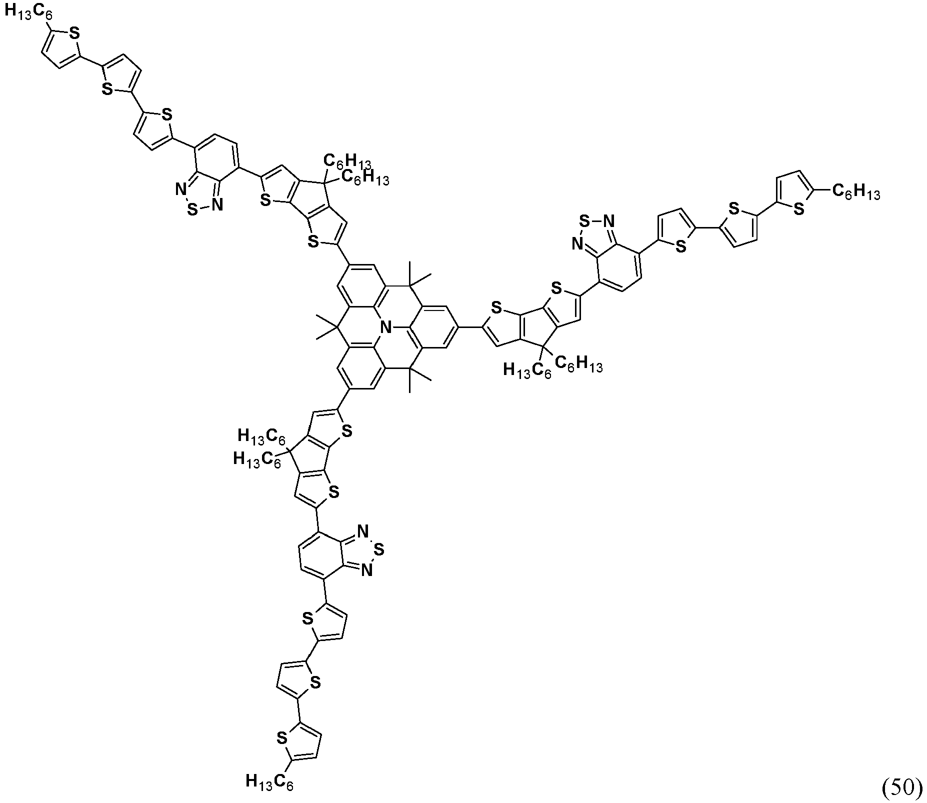

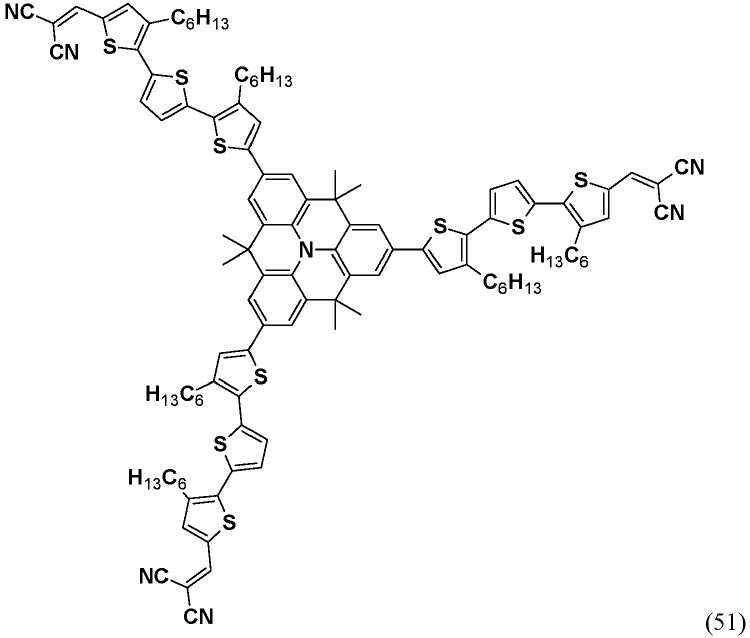

The invention also provides in another aspect a photovoltaic solid state device comprising a compound of formula (I) and/or formula (II).

The photovoltaic solid state device is selected from a solar cell, a heterojunction, an optoelectronic device, a light emitting device. Preferably the solar cell of the invention is a solid state solar cell. Preferably the heterojunction is a solid heterojunction.

According to an embodiment, the photovoltaic device of the invention comprises a conducting support layer, a surface-increasing scaffold structure, a sensitizer or sensitizer layer, a hole transporting layer and a counter electrode and/or metal layer.
In an embodiment, the hole transporting layer of the photovoltaic device is a compound of formula (I) and/or of formula (II).

According to an embodiment, the conducting support layer, the scaffold structure, the perovskite layer and the counter electrode are present in this order from one side to the other of the solar cell of the invention. A protective layer may or may not be present, for example at appropriate positions between the above layers, as disclosed elsewhere in this specification.

According to another embodiment, the photovoltaic device comprises a hole collector layer, a conductive layer, an electron blocking layer, a sensitizer layer and a current collector layer, wherein the hole collector layer is coated by the conductive layer; wherein the electron blocking layer is between the conductive layer and the sensitizer layer, which is in contact with the current collector layer being a metal or a conductor.

According to a further embodiment, the conductive material is selected from one or more conductive polymers or one or more hole transporting materials, which may be selected from poly(3,4-ethylenedioxythiophene):poly(styrenesulfonate) (PEDOT:PSS), poly(3,4-ethylenedioxythiophene):poly(styrenesulfonate):grapheme nanocomposite (PEDOT:PSS:graphene), poly(N-vinylcarbazole) (PVK) and sulfonated poly(diphenylamine) (SPDPA), preferably from PEDOT:PSS, PEDOT:PSS:graphene and PVK, more preferably from PEDOT:PSS. Conductive polymers may also be selected from polymers comprising polyaniline, polypyrrole, polythiophene, polybenzene, polyethylenedioxythiophene, polypropylenedioxy-thiophene, polyacetylene, and combinations of two or more of the aforementioned, for example. The conductive polymer of the invention is preferably selected from the above polymer in a watery dispersion.

For the purpose of the present specification, the expression "in electric contact with" means that electrons or holes can get from one layer to the other layer with which it is in electric contact, at least in one direction. In particular, considering the electron flow in the operating device exposed to electromagnetic radiation, layers through which electrons and/or holes are flowing are considered to be in electric contact. The expression "in electric contact with" does not necessarily mean, and preferably does not mean, that electrons and/or holes can freely move in any direction between the layers.

The conducting support layer is preferably substantially transparent. "Transparent" means transparent to at least a part, preferably a major part of the visible light. Preferably, the conducting support layer is substantially transparent to all wavelengths or types of visible light. Furthermore, the conducting support layer may be transparent to non-visible light, such as UV and IR radiation, for example.

According to an embodiment, the conducting support layer provides the support layer of the solar cell of the invention. Preferably, the solar cell is built on said support layer. According to another embodiment, the support of the solar cell is provided on the side of the counter electrode. In this case, the conductive support layer does not necessarily provide the support of the device, but may simply be or comprise a current collector, for example a metal foil.

The conducting support layer preferably functions and/or comprises a current collector, collecting the current obtained from the solar cell. The conducting support layer may comprise a material selected from indium doped tin oxide (ITO), fluorine doped tinoxide (FTO), ZnO-Ga₂O₃, ZnO-Al₂O₃, tin-oxide, antimony doped tin oxide (ATO), SrGeO₃ and zinc oxide, preferably coated on a transparent substrate, such as plastic or glass. In this case, the plastic or glass provides the support structure of the layer and the cited conducting material provides the conductivity. Such support layers are generally known as conductive glass and conductive plastic, respectively, which are thus preferred conducting support layers in accordance with the invention. According to an embodiment, the conducting support layer comprises a conducting transparent layer, which may be selected from conducting glass and from conducting plastic.

According to an embodiment of the invention, a surface-increasing scaffold structure is provided on said conducting support structure or on a protective layer that may be provided on said scaffold structure.

According to an embodiment of the solar cell and the heterojunction of the invention, the surface-increasing scaffold structure is nanostructured and/or nanoporous. The scaffold structure is thus preferably structured on a nanoscale. The structures of said scaffold structure increase the effective surface compared to the surface of the conductive support.

According to an embodiment, said scaffold structure is made from and/or comprises a metal oxide. For example, the material of the scaffold structure is selected from semiconducting materials, such as Si, TiO₂, SnO₂, Fe₂O₃, ZnO, WO₃, Nb₂O₅, CdS, ZnS, PbS, Bi₂S₃, CdSe, CdTe, SrTiO₃, GaP, InP, GaAs, CuInS₂, CuInSe₂, and combinations thereof, for example. Preferred semiconductor materials are Si, TiO₂, SnO₂, ZnO, WO₃, Nb₂O₅ and SrTiO₃, for example. According to an embodiment, the surface-increasing scaffold structure is nanostructured and/or nanoporous.

The invention does not intend to exclude the possibility that there are one or more intermediate layers between the scaffold structure and the conductive support. Such intermediate layers, if present, would preferably be conducting and/or semiconducting.

According to an embodiment, the sensitizer layer of the photovoltaic device comprising at least one pigment being selecting from organic, inorganic, organometallic and organic-inorganic pigments or a combination thereof. The sensitizer is preferably a light absorbing compound or material. Preferably, the sensitizer is a pigment, and most preferably the sensitizer is an organic-inorganic pigments.

The sensitizer layer may comprise one or more pigments of the group consisting of organometallic sensitizing compounds (telocyanine derived compounds, porphyrine derived compounds), metal free organic sensitizing compounds (diketopyrrolopyrrole (DPP) based sensitizer), inorganic sensitizing compounds such as quantum dots, Sb₂S₃ (Antimonysulfide, for example in the form of thin films), aggregates of organic pigments, nanocomposites, in particular organic-inorganic perovskites, and combinations of the aforementioned. For the purpose of the invention, it is in principle possible to use any type of dyes or sensitizer, including combinations of different types of dyes or different dyes of the same type.

According to one embodiment, the photovoltaic device of the invention comprising a compound of formula (I) and/or of formula (II) further comprises an organic-inorganic perovskite as sensitizer, said perovskite being under the form of layer.

According to an embodiment, the sensitizer layer of the photovoltaic device of the invention is coated by a layer comprising the compound of formula (I) and/or of formula (II). Preferably said sensitizer layer comprises an organic-inorganic perovskite.

According to an embodiment, the sensitizer or the sensitizer layer comprises, consists of or is made of an organic-inorganic perovskite. Said organic-inorganic perovskite is provided under a film of one perovskite pigment or mixed perovskite pigments or perovskite pigments mixed with further dyes or sensitizers.

According to a further embodiment, the sensitizer layer comprises a further pigment in addition to the organic-inorganic perovskite pigment, said further pigment selected from organic pigment, organometallic pigment or inorganic pigment.

Organometallic sensitizers are disclosed, for example, in EP0613466, EP0758337, EP 0983282, EP 1622178, WO2006/038823, WO2009/107100, WO2010/055471 and WO2011/039715. Exemplary organic dyes are those disclosed in WO2009/098643, EP1990373, WO2007/100033 for example. An organic dye was also used in European patent application no. EP11161954.0. and in PCT/IB2011/054628. Metal free organic sensitizers such as DPP based compounds are disclosed, for example, in PCT/IB2013/056648 and in European patent application no. EP12182817.2.

The term "perovskite", for the purpose of this specification, refers to the "perovskite structure" and not specifically to the perovskite material, CaTiO3. For the purpose of this specification, "perovskite" encompasses and preferably relates to any material that has the same type of crystal structure as calcium titanium oxide and of materials in which the bivalent cation is replaced by two separate monovalent cations. The perovskite structure has the general stoichiometry AMX₃, where "A" and "M" are cations and "X" is an anion. The "A" and "M" cations can have a variety of charges and in the original Perovskite mineral (CaTiO₃), the A cation is divalent and the M cation is tetravalent. For the purpose of this invention, the perovskite formulae includes structures having three (3) or four (4) anions, which may be the same or different, and/or one or two (2) organic cations, and/or metal atoms carrying two or three positive charges, in accordance with the formulae presented elsewhere in this specification.

According to an embodiment, the photovoltaic device of the invention comprises one or more layer of an organic-inorganic perovskite.

According to an embodiment, the sensitizer layer comprises, consists essentially of or consists of a nanocomposite material or an organic-inorganic pigments. According to a preferred embodiment, the sensitizer layer comprises, consists essentially of or consists of an organic-inorganic perovskite.

According to a further embodiment, the organic-inorganic perovskite layer material of the photovoltaic device of the invention further comprises a perovskite-structure of any one of formulae (I), (II), (III), (IV), (V) and/or (VI) below:

| | |
|---|---|
| AA'MX4 | (I) |
| AMX3 | (II) |
| AA'N2/3X4 | (III) |
| AN2/3X3 | (IV) |
| BN2/3X4 | (V) |
| BMX4 | (VI) |

wherein,
- A and A' are organic, monovalent cations that are independently selected from primary, secondary, tertiary or quaternary organic ammonium compounds, including N-containing heterorings and ring systems, A and A' having independently from 1 to 60 carbons and 1 to 20 heteroatoms;
- B is an organic, bivalent cation selected from primary, secondary, tertiary or quaternary organic ammonium compounds having from 1 to 60 carbons and 2-20 heteroatoms and having two positively charged nitrogen atoms;
- M is a divalent metal cation selected from the group consisting of Cu²⁺, Ni²⁺, Co²⁺, Fe²⁺, Mn²⁺, Cr²⁺, Pd²⁺, Cd²⁺, Ge²⁺, Sn²⁺, Pb²⁺, Eu²⁺, or Yb²⁺;
- N is selected from the group of Bi³⁺ and Sb³⁺; and,
- X is independently selected from Cl⁻, Br⁻, I⁻, NCS⁻, CN⁻, and NCO⁻.

In particular, the three or four X may be the same or different. For example, in AMX₃ (formula II) may be expressed as formula (II') below:

| | |
|---|---|
| AMXⁱXⁱⁱXⁱⁱⁱ | (II') |

wherein Xⁱ, Xⁱⁱ, Xⁱⁱⁱ are independently selected from Cl⁻, Br⁻, I⁻, NCS⁻, CN⁻, and NCO⁻, preferably from halides (Cl⁻, Br⁻, I⁻), and A and M are as defined elsewhere in this specification. Xⁱ, Xⁱⁱ, Xⁱⁱⁱ may thus be the same or different in this case. The same principle applies to the perovskites of formulae (I) and (III)-(VI) and the more specific embodiments of formulae (VIII) to (XIV) below. In case of AA'MX₄ (formula I), for example, formula (I') applies:

| | |
|---|---|
| AA'M XⁱXⁱⁱXⁱⁱⁱ X^{iv} | (I') |

wherein Xⁱ, Xⁱⁱ, Xⁱⁱⁱ are independently selected from Cl⁻, Br⁻, I⁻, NCS⁻, CN⁻, and NCO⁻, preferably from halides (Cl⁻, Br⁻, I⁻).

Preferably, if Xⁱ, Xⁱⁱ, Xⁱⁱⁱ in formulae (II) and (IV) or Xⁱ, Xⁱⁱ, Xⁱⁱⁱ, X^{iv} in formulae (I), (III), (V) or (VI) comprise different anions X, there are not more than two different anions. For example, Xⁱ and Xⁱⁱ being the same with Xⁱⁱⁱ being an anion that is different from Xⁱ and Xⁱⁱ.

According to a preferred embodiment, the perovskite material has the structure selected from one or more of formulae (I) to (III), preferably (II) or (II').

According to a preferred embodiment, said organic-inorganic perovskite layer comprises a perovskite-structure of any one of the formulae (VIII) to (XIV):

| | |
|---|---|
| APbX₃ | (VIII) |
| ASnX₃ | (IX) |
| ABiX₄ | (X) |
| AA'PbX₄ | (XI) |
| AA'SnX₄ | (XII) |
| BPbX₄ | (XIII) |
| BSnX₄ | (XIV) |

wherein A, A', B and X are as defined elsewhere in this specification. Preferably, X is preferably selected from Cl⁻, Br⁻ and I⁻, most preferably X is I⁻.

According to a preferred embodiment, said organic-inorganic perovskite layer comprises a perovskite-structure of the formulae (VIII) to (XII), more preferably (VIII) and/or (IX) above.

According to an embodiment, A and A' are monovalent cations selected independently from any one of the compounds of formulae (28) to (35) below: 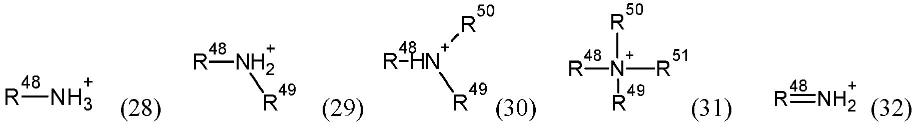 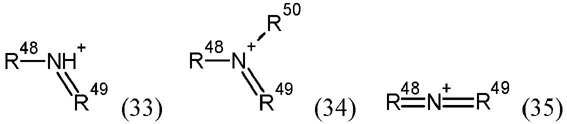 wherein,
any one of R⁴⁸, R⁴⁹, R⁵⁰ and R⁵¹ is independently selected from C1-C15 organic substituents comprising from 0 to 15 heteroatoms.

According to an embodiment of said C1-C15 organic substituent any one, several or all hydrogens in said substituent may be replaced by halogen and said organic substituent may comprise up to fifteen (15) N, S or O heteroatoms, and wherein, in any one of the compounds (29) to (35), the two or more of substituents present (R⁴⁸, R⁴⁹, R⁵⁰ and R⁵¹, as applicable) may be covalently connected to each other to form a substituted or unsubstituted ring or ring system. Preferably, in a chain of atoms of said C1-C15 organic substituent, any heteroatom is connected to at least one carbon atom. Preferably, neighboring heteroatoms are absent and/or heteroatom-heteroatom bonds are absent in said C1-C15 organic substituent comprising from 0 to 15 heteroatoms. The heteroatoms may be selected from N, S, and/or O.

According to an embodiment any one of R⁴⁸, R⁴⁹, R⁵⁰ and R⁵¹ is independently selected from C1 to C15 aliphatic and C4 to C15 aromatic or heteroaromatic substituents, wherein any one, several or all hydrogens in said substituent may be replaced by halogen and wherein, in any one of the compounds (29) to (35), the two or more of the substituents present may be covalently connected to each other to form a substituted or unsubstituted ring or ring system.

According to an embodiment, B is a bivalent cation selected from any one of the compounds of formulae (36) and (37) below:

R₄₈-G-R₄₉ (36)

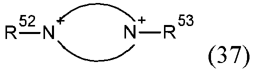

wherein,
in the compound of formula (36), G is an organic linker structure having 1 to 10 carbons and 0 to 5 heteroatoms selected from N, S, and/or O, wherein one or more hydroge atoms in said G may be replaced by halogen;
wherein any one of R₄₈ and R₄₉ is independently selected from any one of the substituents (38) to (43) below: 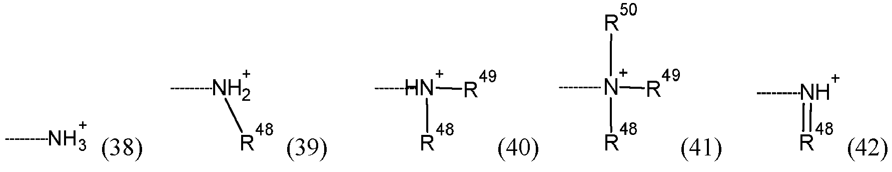 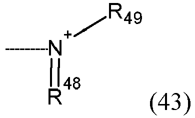
wherein the dotted line in the substituents (38) to (43) represents the bond by which said substituent is connected to the linker structure G;
wherein R⁴⁸, R⁴⁹, and R⁵⁰ are independently as defined above with respect to the compounds of formulae (28) to (35);
wherein R₄₈ and R₄₉, if they are both different from substituent (38), may be covalently connected to each other by way of their substituents R⁴⁸, R⁴⁹, and/or R⁵⁰, as applicable, and wherein any one of R⁴⁸, R⁴⁹, and R⁵⁰, if present, may be covalently connected to G or the ring structure of compound (36), independently from whether said substituent is present on R₄₈ or R₄₉;
and wherein, in the compound of formula (37), the circle containing said two positively charged nitrogen atoms represents a substituted or unsubstituted aromatic ring or ring system comprising 4 to 15 carbon atoms and 2 to 7 heteroatoms or 4 to 10 carbon atoms and 2 to 5 heteroatoms, wherein said nitrogen atoms are ring heteroatoms of said ring or ring system, and wherein the remaining of said heteroatoms may be selected independently from N, O and S and wherein R⁵² and R⁵³ are independently selected from H and from substituents as R⁴⁸ to R⁵¹. Halogen atom substituting hydrogen atom totally or partially may also be present in addition to and/or independently of said 2 to 7 heteroatoms.

Preferably, if the number of carbons is in G is impair, the number of heteroatoms is smaller than the number of carbons. Preferably, in the ring structure of formula (37), the number of ring heteroatoms is smaller than the number of carbon atoms. According to an embodiment, G is an aliphatic, aromatic or heteroaromatic linker structure having from 1 to 10 carbons.

Preferably, the dotted line in substituents (38) to (43) represents a carbon-nitrogen bond, connecting the nitrogen atom shown in the substituent to a carbon atom of the linker.

According to an embodiment, in the compound of formula (36), G is an organic linker structure having 1 to 8 carbons and from 0 to 4 N, S and/or O heteroatoms or having 1 to 6 carbons and from 0 to 3 N, S and/or O heteroatoms, wherein any one, several or all hydrogens in said G may be replaced by halogen. Preferably, L is an aliphatic, aromatic or heteroaromatic linker structure having 1 to 8 carbons, wherein any one, several or all hydrogens in said G may be replaced by halogen. According to an embodiment, in the compound of formula (36), said linker G is free of any O or S heteroatoms. According to an embodiment, G is free of N, O and/or S heteroatoms.

According to an embodiment, any one of R⁴⁸, R⁴⁹, R⁵⁰ and R⁵¹ is independently selected from C1 to C10 alkyl, C2 to C10 alkenyl, C2 to C10 alkynyl, C4 to C10 heteroaryl and C6 to C10 aryl, wherein said alkyl, alkenyl, and alkynyl, if they comprise 3 or more carbons, may be linear, branched or cyclic, wherein said heteroaryl and aryl may be substituted or unsubstituted, and wherein several or all hydrogens in R¹-R⁴ may be replaced by halogen.

According to an embodiment, any one of R⁴⁸, R⁴⁹, R⁵⁰ and R⁵¹ is independently selected from C1 to C8 alkyl, C2 to C8 alkenyl, C2 to C8 alkynyl, C4 to C8 heteroaryl and C6 to C8 aryl, wherein said alkyl, alkenyl, and alkynyl, if they comprise 3 or more carbons, may be linear, branched or cyclic, wherein said heteroaryl and aryl may be substituted or unsubstituted, and wherein several or all hydrogens in R¹-R⁴ may be replaced by halogen.

According to an embodiment, any one of R⁴⁸, R⁴⁹, R⁵⁰ and R⁵¹ is independently selected from C1 to C6 alkyl, C2 to C6 alkenyl, C2 to C6 alkynyl, C4 to C6 heteroaryl and C6 aryl, wherein said alkyl, alkenyl, and alkynyl, if they comprise 3 or more carbons, may be linear, branched or cyclic, wherein said heteroaryl and aryl may be substituted or unsubstituted, and wherein several or all hydrogens in R¹-R⁴ may be replaced by halogen.

According to an embodiment, any one of R⁴⁸, R⁴⁹, R⁵⁰ and R⁵¹ is independently selected from C1 to C4 alkyl, C2 to C4 alkenyl and C2 to C4 alkynyl, wherein said alkyl, alkenyl and alkynyl, if they comprise 3 or more carbons, may be linear, branched or cyclic, and wherein several or all hydrogens in in R¹-R⁴ may be replaced by halogen.

According to an embodiment, any one of R⁴⁸, R⁴⁹, R⁵⁰ and R⁵¹ is independently selected from C1 to C3, preferably C1 to C2 alkyl, C2 to C3, preferably C2 alkenyl and C2 to C3, preferably C2 alkynyl, wherein said alkyl, alkenyl and alkynyl, if they comprise 3 or more carbons, may be linear, branched or cyclic, and wherein several or all hydrogens in R¹-R⁴ may be replaced by halogen.

According to an embodiment, any one of R⁴⁸, R⁴⁹, R⁵⁰ and R⁵¹ is independently selected from C1 to C4, more preferably C1 to C3 and even more preferably C1 to C2 alkyl. Most preferably, any one of R¹, R², R³ and R⁴ are methyl. Again, said alkyl may be completely or partially halogenated.

According to an embodiment, A, A' and B are monovalent (A, A') and bivalent (B) cations, respectively, selected from substituted and unsubstituted C5 to C6 rings comprising one, two or more nitrogen heteroatoms, wherein one (for A and A') or two (for B) of said nitrogen atoms is/are positively charged. Substituents of such rings may be selected from halogen and from C1 to C4 alkyls, C2 to C4 alkenyls and C2 to C4 alkynyls as defined above, preferably from C1 to C3 alkyls, C3 alkenyls and C3 alkynyls as defined above. Said ring may comprise further heteroatoms, which may be selected from O, N and S. Bivalent organic cations B comprising two positively charged ring N-atoms are exemplified, for example, by the compound of formula (37) above. Such rings may be aromatic or aliphatic.

A, A' and B may also comprise a ring system comprising two or more rings, at least one of which being from substituted and unsubstituted C5 to C6 ring as defined as above. The elliptically drawn circle in the compound of formulae (10) may also represent a ring system comprising, for example, two or more rings, but preferably two rings. Also if A and/or A' comprises two rings, further ring heteroatoms may be present, which are preferably not charged, for example.

According to an embodiment, however, the organic cations A, A' and B comprise one (for A, A'), two (for B) or more nitrogen atom(s) but are free of any O or S or any other heteroatom, with the exception of halogens, which may substitute one or more hydrogen atoms in cation A and/or B.

A and A' preferably comprise one positively charged nitrogen atom. B preferably comprises two positively charged nitrogen atoms.

A, A' and B may be selected from the exemplary rings or ring systems of formulae (44) and (45) (for A) and from (46) to (48) (for B) below: 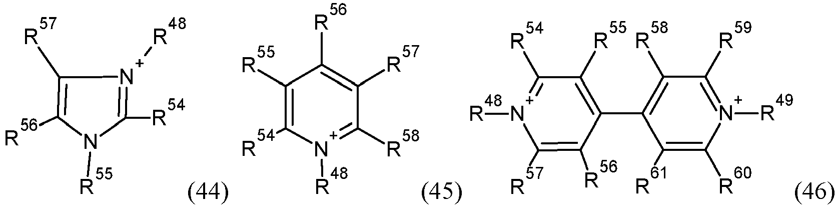 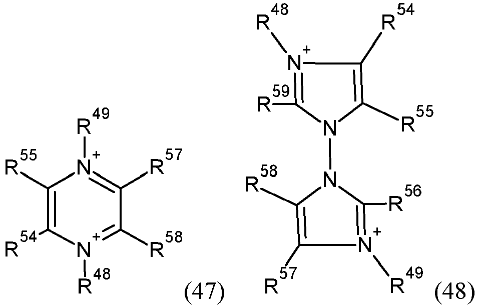 in which R⁴⁸ and R⁴⁹ are, independently, as defined above, and R⁵⁴, R⁵⁵, R⁵⁶, R⁵⁷, R⁵⁸, R⁵⁹ and R⁶⁰ are independently selected from H, halogen and substituents as defined above for R⁴⁸ to R⁵¹. Preferably, R⁵⁴, R⁵⁵, R⁵⁶, R⁵⁷, R⁵⁸, R⁵⁹ and R⁶⁰ are selected from H and halogen, most preferably H.

In the organic cations A, A' and B, hydrogen atoms may be substituted by halogens, such as F, Cl, I, and Br, preferably F or Cl. Such a substitution is expected to reduce the hygroscopic properties of the perovskite layer or layers and may thus provide a useful option for the purpose of the present specification.

According to a preferred embodiment, A and A' are independently selected from organic cations of formula (28). Preferably, R⁴⁸ in the cation of formula (28) is selected from C1 to C8 organic substituents comprising, from 0 to 4 N, S and/or O heteroatom. More preferably, R¹ is selected from C1 to C4, preferably C1 to C3 and most preferably C1 to C2 aliphatic substituents.

According to a preferred embodiment, the metal M is selected from Sn²⁺ and Pb²⁺, preferably Pb²⁺. According to a preferred embodiment, N is Sb³⁺.

According to a preferred embodiment, the three or four X are independently selected from Cl⁻, Br⁻, and I⁻.

According to a preferred embodiment, the organic-inorganic perovskite material has the formula of formulae (XV) to (XIX) below:

| | |
|---|---|
| AMI₃ | (XV) |
| AMI₂Br | (XVI) |
| AMI₂Cl | (XVII) |
| AMBr₃ | (XVII) |
| AMCl₃ | (XIX) |

wherein A and M are as defined elsewhere in this specification, including the preferred embodiments of A and M, such as those defined below. Preferably, M is selected from Sn²⁺ and Pb²⁺. Preferably, A is selected from organic cations of formula (28). Preferably, R⁴⁸ in the cation of formula (28) is selected from C1 to C8 organic substituents comprising, from 0 to 4 N, S and/or O heteroatom. More preferably, R⁴⁸ is selected from C1 to C4, preferably C1 to C3 and most preferably C1 to C2 aliphatic substituents.

According to a preferred embodiment, the organic-inorganic perovskite is a compound of formula (VII) (AMXiXiiXiii), wherein A is a monovalent cation of formula (1) as defined above, M is Sn²⁺ or Pb²⁺, and Xi, Xii, Xiii are independently selected from Cl⁻, Br⁻, I⁻. Preferably, R¹ in the cation of formula (1) is selected from C1 to C4, preferably C1 to C3 and most preferably C1 to C2 aliphatic substituents. Preferably, Xi -Xiii are identical.

In the moieties of formula (1) to (48), the connection of any moiety to quinozilino acridine core, a preceding moiety or a following moiety (for example, if one or the other of the integer m, n is different from 0) is illustrated by way of a dashed line representing the bond indicating the connection between said moieties.

In a further aspect, the invention provides a use of a compound of formula (I) and/or of formula (II) as a hole transporting and light absorbing material in photovoltaic solid state device.

The present invention will now be illustrated by way of examples. These examples do not limit the scope of this invention, which is defined by the appended claims.

### Examples

### Example 1 Synthesis of compound of formula (49) (Fused F)

### Tris[[4-[3,3'-dihexylsilylene-2,2'-bithiophene]-7-[5"-n-hexyl-(2, 2';5',2"-terthiophene)-5-yl]-benzo[c]-[1,2,5]thiadiazole]-2,6,10-yl]-4,4,8,8,12,12-Hexamethyl-4H,8H,12Hbenzo[1,9]quinolizino[3,4,5,6,7,-defg]acridine

The scheme of synthesis of compound of formula (49), a low band gap highly absorbing HTM is shown in Figure 1A.

Under nitrogen atmosphere and at -78 °C, *n*-BuLi (0.21 ml, 1.6 M in hexane) was added drop-wise to a anhydrous THF solution containing 2,6,10-tribromo-4,4,8,8,12,12-Hexamethyl-4H,8H,12Hbenzo[1,9]quinolizino[3,4,5,6,7,-defg]acridine (v) (0.06 g, 0.1 mmol). After 1 hour stirring at -78 °C, 0.33 ml of trimethyltin chloride (1.0 M in THF solution) was added slowly to the reaction solution at -78 °C. The temperature of the solution was warmed to room temperature and the reaction was stirred for overnight. Then, the reaction was quenched with brine. The solution was extracted with dichloromethane, dried with MgSO₄. 2,6,10-trimethylstannyl-4,4,8,8,12,12-Hexamethyl-4H,8H,12Hbenzo[1,9]quinolizino[3,4,5,-6,7,-defg]acridine (**vi**) was obtained without any purification. 4-[5-Bromo-3,3'-dihexylsilylene-2,2'-bithiophene]-7-[5"-n-hexyl-(2,2';5',2"-terthiophene)-5-yl]- benzo[c]-[1,2,5] thiadiazole (**iv**) (0.36 g, 0.4 mmol), 2,6,10-trimethylstannyl-4,4,8,8,12,12-Hexamethyl-4H,8H,12H-benzo[1,9]quinolizino[3,4,5,6,7,-defg]acridi-ne (**vi**) (0.085 g, 0.1 mmol), Pd(PPh₃)₄ (0.012 g, 0.01 mmol), and anhydrous toluene (50 ml) were added to a 125 mL flame-dried 2-neck round-bottom flask with a condenser under a nitrogen atmosphere. The reaction mixture was heated to reflux for 2 days. The reaction mixture was then cooled to room temperature. The organic layer was separated and dried over anhydrous magnesium sulfate. The solvent was removed in *vacuo.* The orange product was purified by column chromatography.
Yield: 60 %. Mp: 97 °C. MS: *m*/*z* 2840 [M+]. 1H NMR (300 MHz, CDC13): *δ* 8.18 (s, 3H), 8.01 (d, *J* = 3.9 Hz, 3H), 7.81 (s, 6H), 7.66 (s, 6H), 7.35 (s, 3H), 7.22 (d, *J* = 3.6 Hz, 3H), 7.16 (d, *J* = 3.6 Hz, 3H), 7.02 (d, *J* = 3.6 Hz, 3H), 6.99 (d, *J* = 3.3 Hz, 3H) 6.69 (d, *J* = 3.9 Hz, 3H), 2.80 (t, 6H), 1.78 (s, 18H), 1.68 - 1.30 (m, 72H), 1.07 (m, 12H), 0.89 (m, 27H). 13C NMR (75 MHz, CDCl3): *δ* 152.55, 152.49, 151.33, 147.61, 146.44, 145.84, 144.28, 142.50, 140.26, 138.46, 140.26, 138.46, 138.21, 137.33, 135.45, 134.52, 130.96, 130.57, 129.68, 126.44, 126.36, 125.43, 124.71, 124.58, 124.07, 121.29, 35.94, 33.18, 31.71, 31.25, 30.40, 29.90, 28.97, 24.47, 22.86, 22.78, 14.48, 14.20, 12.28.

### Example 2. Photovoltaic characterization of compound of formula (49) (Fused-F)

A 450 W xenon lamp (Oriel) with a Schott K133 Tempax sunlight filter was used as the light source. The current-voltage characteristics were measured by applying an external potential bias to the device, and recorded the generated photocurrent with a Keithley model 2400 digital source meter. IPCE spectra were measured by an array of white light emitting diodes. The excitation beam passed through a Genimi-180 double monochromator (Jobin Yvon Ltd) and chopped at approximately 2 Hz before it illuminated the device. The spectra were recorded using a Model SR830 DSP Lock-In Amplifier. In both cases, the device was measured by using a black mask with an area of 0.2025 cm².

The UV-Visible absorption and emission spectra of compound of formula (49) (Fused-F) in solution are shown in Figure 2A. In contrast to the most efficient HTMs spiro-MeOTAD and PTAA, which exhibit absorptions mainly in the UV region, said compound of formula (49) shows intensive charge-transfer absorption bands in the visible region with peak at 421 nm (molar extinction coefficients ε = 108000 L mol⁻¹ cm⁻¹) and 580 nm (ε = 135000 L mol⁻¹ cm⁻¹). The visible light harvesting ability of the compound of formula (49) serves the purpose of absorbing the remaining light passed through the CH₃NH₃PbI₃ layer. The fluorescence spectrum shows a maximum at 746 nm, with a large Stokes shift of more than 150 nm. To further investigate the contribution of light absorption from compound of formula (49), the UV-is spectra of CH₃NH₃PbI₃ films with and without compound of formula (49) were recorded for comparison. As seen in Figure 2B, with the presence of compound of formula (49), there is a significant enhancement in absorption in the whole visible region for the perovskite film, confirming its additional contribution for light harvesting. It absorbs the photons after passing through the CH₃NH₃PbI₃ layer, and contributes for the photocurrent generation of the device.

### Example 3. Characterization of solar cell comprising organic-inorganic perovskite CH₃NH₃PbI and compound of formula (49) (Fused-F) as HTM

### Solar Cell Fabrication

CH₃NH₃PbI was synthesized as reported in Etgar et al. ((2012), J. Am. Chem. Soc. 134, 17396-17399). The compact TiO₂ layer was deposited on the etched fluorine-doped tin oxide conductive glass (NSG 10) by spray pyrolysis at 450°C, using titanium diisopropoxide bis(acetylacetonate) solution as precursor and O₂ as carrier gas. The substrate was then dipped into a 20 mM TiCl₄ aqueous solution for 30 min at 70°C, and sintered at 500°C for 30 min. The mesoporous TiO₂ film was spin-coated on the top at 5000 rpm for 30 s by using Dyesol 18NRT TiO₂ paste, following by sintering at 500°C for 30 min in air. The 1.3 M PbI₂ in DMF solution was dropped on the TiO₂ surface, and spin-coated at 6500 rpm for 30 s in the dry air box. The film was annealed at 70 °C for 30 min. After cooling down, the film was dipped into CH₃NH₃I solution (10 mg / mL in 2-propanol) for 25 s, and dried at 70 °C for 15 min. The 12 mM Fused-F in chlorobenzene was spin-coated on the top of the perovskite layer with the spin speed of 3000 rpm. Finally, 60 nm of Au was deposited by thermal evaporation on the top of the HTM as the back contact.

Figure 1B shows the device architecture where fluorine-doped tin oxide glass substrate coated with a thin compact TiO₂ as a hole blocking layer. A mesoporous TiO₂ layer of about 250 nm as scaffold as well as electron collector was deposited. Lead iodide (PbI₂) was deposited onto the mesoporous TiO₂ surface by spin-coating, followed by immersion into a solution of methylammonium iodide (CH₃NH₃I) to form perovskite. The Fused-F HTM is then introduced by spin-coating from the chlorobenzene solution. No additives are used, such as lithium bis(trifluoromethyl sulfonyl)imide (LiTFSI), 4-tert-butylpyridine (TBP) and the dopants, which were normally used together with spiro-MeOTAD and semiconducting polymers to get higher efficiencies. We measured cyclic voltammetry of the Fused-F, and found that the highest occupied molecular orbital (HOMO) and lowest unoccupied molecular orbital (LUMO) energy levels are -5.23 eV and -3.66 eV, respectively. Under illumination, free charge carriers generated in the CH₃NH₃PbI₃ layer, and can be extracted by both electron transfer to TiO₂ and hole transfer to Fused-F due to their appropriate energy levels to avoid unfavorable recombination.

Surface morphology and film thickness were examined using a high-resolution scanning electron microscope (SEM). The ∼100 nm film of CH₃NH₃PbI₃ nanocrystals is uniformly distributed over the surface of titanium dioxide photoanode. Further CH₃NH₃PbI₃ nanocrystals are uniformly covered with fused-F. In addition to the infiltration into the perovskite layer, the fused-F forms an overlayer.

The current-voltage (*J*-*V*) characteristics of TiO₂/CH₃NH₃PbI₃/Fused-F/Au solar cell measured under dark and simulated AM 1.5 G (100 mW cm⁻²) irradiation is shown in Figure 3A, and the corresponding photovoltaic parameters are summarized in Table 1. For comparison, a reference cell without a HTM was fabricated.

**Table 1 Photovoltaic parameters derived from J-V measurements of CH₃NH₃PbI₃ based devices with and without Fused-F (compound of formula (49)**

| | ***J*_{SC} [mA cm⁻²]** | ***V*_{OC} [mV]** | ***FF*** | **PCE [%]** |
|---|---|---|---|---|
| **Compound (49) (Fused-F)** | 17.9 | 1036 | 0.68 | 12.8 |
| **No HTM** | 14.0 | 790 | 0.69 | 7.6 |

The device with Fused-F shows a short-circuit current density (*J_{sc}*) of 17.9 mA/cm², an open-circuit voltage (*V*_{oc}) of 1036 mV and a fill factor (*FF*) of 0.68, leading to a PCE of 12.8%. Under the same conditions, the device without HTM shows both lower *J*_{sc} and *V*_{oc}, finally giving a PCE of 7.6%. The high *J*_{sc} with the presence of Fused-F is due to the effective charge extraction, as well as the improved light harvesting. The incident photo-to-current conversion efficiency (IPCE) spectra for the cells are given in Figure 3B. The presence of Fused-F showed improvement of the photocurrent in the whole visible region between 400 to 800 nm, further confirming the efficient charge extraction.

In summary, the novel hole transporting and light absorbing material of the invention, a compound of formula (I) and/or formula (II), more specifically compound of formula (49) shows suitable homo levels to extract efficiently holes from perovskite and contributes in enhancing light harvesting efficiency. The Fused-F (compound of formula (49)) is a hybrid molecule that has donor and acceptor functionalities to induce charge transfer-transitions with the molecule. The highest occupied molecular orbitals energy levels were modulated incorporating thiophene groups and the solubility is due to presence of long alkyl groups.

### Example 5. Synthesis of compound of formula (51) (FA-CN-Final)

### 2,6,10-tris-(3,3',3"',4'-tetraoctyl[2,2':5',2":5",2"'-quaterthiophen]-5"'-methylenemalononitrile-5-yl)-4,4,8,8,12,12-Hexamethyl-4H,8H,12H-benzo[1,9]quinolizino[3,4,5,6,7,-defg]acridine

The scheme of synthesis of compound of formula (51) is shown in Figure 4A. Compound 3 (0.15 g, 0.088 mmol) and malononitrile (58 mg, 0.88 mmol) was dissolved in dry CHCl₃ and then a few drops of triethylamine was stirred for 1 h, under nitrogen at room temperature. The solution was extracted with dichloromethane and dried over anhydrous magnesium sulfate. The solvent was removed in vacuo. The product was purified by column chromatography. Yield: 80 %. MS: *m*/*z* 1836 [M⁺]. ¹H NMR (300 MHz, CDCl₃): *δ* 7.70 (s, 3H), 7.61 (s, 6H), 7.55 (s, 3H), 7.37 (d, *J* = 3.6 Hz, 3H), 7.20 (d, *J* = 3.9 Hz, 3H), 7.18 (s, 3H), 2.86 (m, 12H), 1.69 (m, 12H), 1.43 - 1.25 (m, 36H), 0.91 (m, 18H). ¹³C NMR (75 MHz, CDC1₃): *δ* 149.94, 144.08, 143.29, 141.92, 140.69, 140.17, 133.23, 132.18, 131.22, 130.62, 128.95, 128.37, 121.28, 114.56, 113.62, 35.94, 33.42, 31.77, 30.77, 30.26, 30.07, 29.55, 22.80, 14.30.

### Example 6. Photovoltaic characterization of compound of formula (51) (FA-CN-Final)

The characterization is performed as described for Example 2.

The UV-Visible absorption and emission spectra of compound of formula (51) (FA-CN-Final) in solution are shown in Figure 4B.

Examples 5 and 6 are reference examples.

## Claims

1. A compound of formula (I): 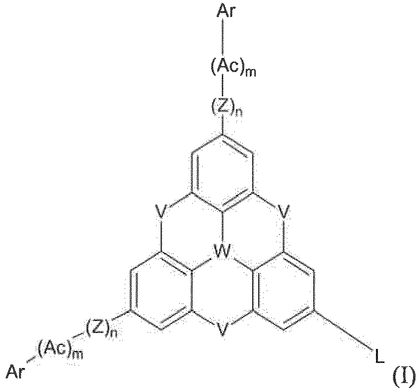 wherein
- W is a nitrogen (N) atom;
- V is, on each occurrence, identically or differently selected from C(R¹)₂, C=O or O, R¹ being selected from C1-C12 alkyl;
- L is selected from moiety of formula ------(Z)ₙ-(Ac)ₘ-Ar as defined below or from moiety of formula (19) 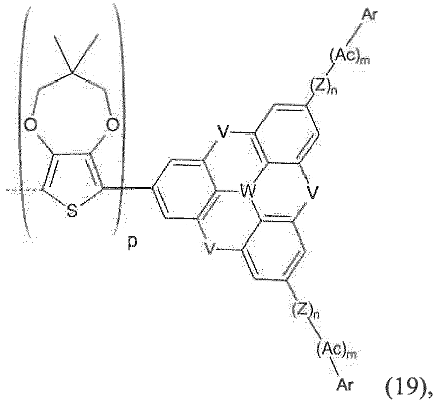 p being 0, 1 or 2 and moiety of formula ------(Z)ₙ-(AC)ₘ-Ar being as defined below;
- Z is a heteroaromatic ring system, on each occurrence, identically or differently selected from C4-C20 heteroaryl and C4-C20 heteroaryloxy group, wherein the heteroatoms are selected from O, S, Se, Si and wherein said heteroaryland heteroaryloxy group are substituted by C1-C20 alkyl, C1-C20 heteroalkyl, C2-C20 alkenyl or C2-C20 alkynyl, wherein said alkyl, alkenyl, and alkynyl, if they comprise 3 or more carbons, may be linear, branched or cyclic, and n is 0, 1, 2, or 3;
- Ac is an acceptor group, on each occurrence, identically or differently selected from a moiety according to any one of the formulae (1) to (18), 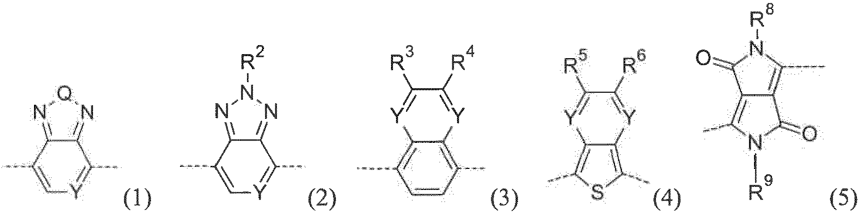 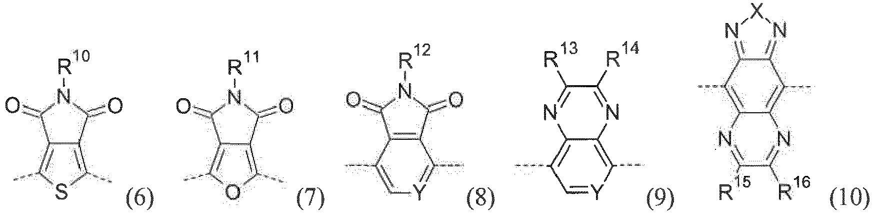 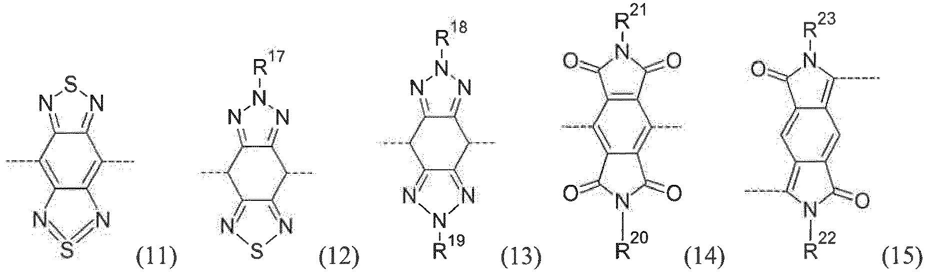 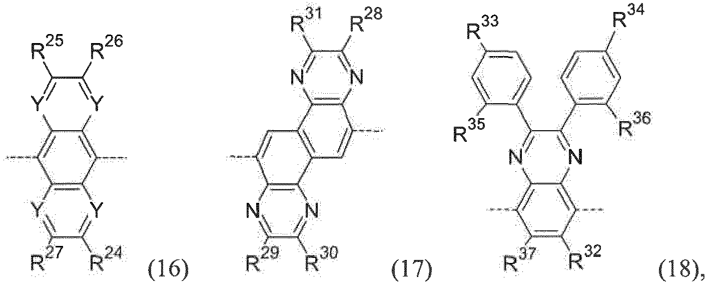
- wherein
- R² to R³⁷ are substituents independently selected from H, ether group, C1-C 16 alkyl, C1-C16 alkoxy group, C1-C16 thioalkyl, C1-C16 alkoxyalkyl, C4-C16 aryl, C1-C16 arylalkyl or C4-C16 heteroaryl, C4-C16 heteroarylalkyl, the heteroatoms being selected from O, S, or N,
- Q is selected from S, O, Se, Si or N and when Q is N, said nitrogen atom is substituted by substituent as defined for R² to R³⁷,
- Y is, on each occurrence, identically or differently selected from N or C, being unsubstituted or substituted by halogen selected from F, Cl, I, and Br, and
- m is 0 or 1;
- -Ar is a successive bound heteroaromatic rings system comprising from 2 to 10 heteroaromatic rings, said heteroaromatic rings being, on each occurrence, identically selected from C4-C20 heteroaryl and C4-C20 heteroaryloxy group, wherein the heteroatoms are selected from O, S, Se, Si and wherein said heteroaryl and heteroaryloxy groups are unsubstituted or substituted by -COOH, C4-C16 cyanoalkenyl carboxylic acid, C1-C20 alkyl, C1-C20 heteroalkyl, C2-C20 alkenyl or C2-C20 alkynyl, wherein said alkyl, alkenyl, and alkynyl, if they comprise 3 or more carbons, may be linear, branched or cyclic.

2. The compound of claim 1, wherein L is moiety of formula ------(Z)ₙ-(Ac)ₘ-Ar and is a compound of formula (II):

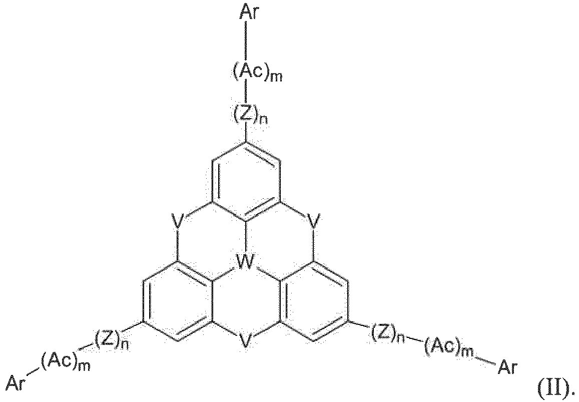

3. The compound according to any one of the preceding claims, wherein Ac is selected from a moiety according to any one of the formulae (1) to (4) and (10) to (13).

4. The compound according to any one of the preceding claims, wherein Ac is selected from a moiety according to any one of the formulae (1), (10) and (11).

5. The compound according to any one of the preceding claims, wherein Q of any one of the formulae (1) to (18) is selected from S, O or Se.

6. The compound according to any one the preceding claims, wherein Y of any one of the formulae (1) to (18) is C.

7. The compound according to any one of the preceding claims, wherein Z is selected from a moiety according to any one of the formulae (20) to (27) 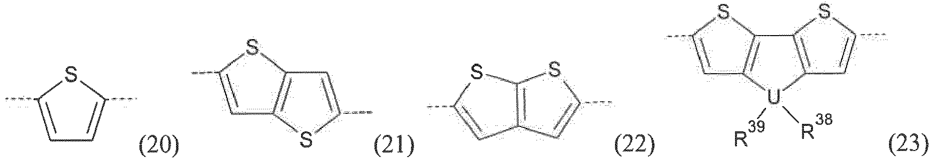 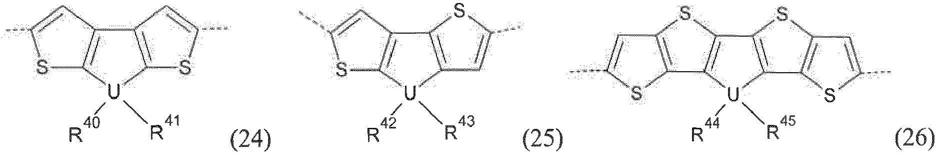 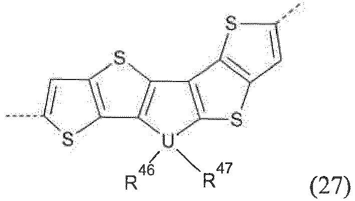
- wherein
- R³⁹ to R⁴⁷ are substituents independently selected from H, C1-C16 alkyl, C1-C16 alkoxy group, C1-C16 thioalkyl, C1-C16 alkoxyalkyl and if they comprise 3 or more carbons, they may be linear or branched;
- U is selected from S, Si or C,

8. The compound according to claim 7, wherein Z is selected from a moiety of any one of the formulae (20), (23) to (26).

9. A photovoltaic solid state device comprising a compound according to any one of claims 1 to 8.

10. The device of claim 9 further comprising an organic-inorganic perovskite as sensitizer, said perovskite being under the form of layer.

11. The device according to claim 10, wherein the organic-inorganic perovskite layer material comprises a perovskite-structure of any one of formulae (I), (II), (III), (IV), (V) and/or (VI) below:
| | |
|---|---|
| AA'MX₄ | (I) |
| AMX₃ | (II) |
| AA'N_{2/3}X₄ | (III) |
| AN_{2/3}X₃ | (IV) |
| BN_{2/3}X₄ | (V) |
| BMX₄ | (VI) |
wherein,
- A and A' are organic, monovalent cations that are independently selected from primary, secondary, tertiary or quaternary organic ammonium compounds, including N-containing heterorings and ring systems, A and A' having independently from 1 to 60 carbons and 1 to 20 heteroatoms;
- B is an organic, bivalent cation selected from primary, secondary, tertiary or quaternary organic ammonium compounds having from 1 to 60 carbons and 2-20 heteroatoms and having two positively charged nitrogen atoms;
- M is a divalent metal cation selected from the group consisting of Cu²⁺, Ni²⁺, Co²⁺, Fe²⁺, Mn²⁺, Cr²⁺, Pd²⁺, Cd²⁺, Ge²⁺, Sn²⁺, Pb²⁺, Eu²⁺, or Yb²⁺;
- N is selected from the group of Bi³⁺ and Sb³⁺; and,
- X is independently selected from Cl⁻, Br⁻, I⁻, NCS⁻, CN⁻, and NCO⁻.

12. The device according to any one of claims 10 to 11, wherein the sensitizer layer made of organic-inorganic perovskite is coated by a layer comprising the compound according to any one of claims 1-8.

13. The device according to any one of claims 9 to 12, wherein said device is selected from a solar cell, a heterojunction, an optoelectronic device and a light emitting device.

14. The device according to any one of claims 9 to 12, wherein the solar cell is a solid state solar cell.

15. Use of a compound according to any one of claims 1-8 as a hole transporting and light absorbing material in photovoltaic solid state device.

## Patentansprüche

1. Verbindung der Formel (I): 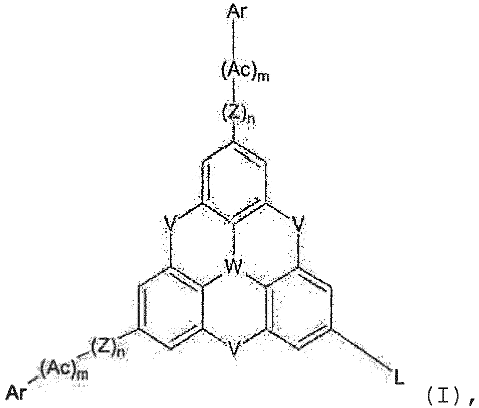 wobei
- W für ein Stickstoffatom (N-Atom) steht;
- V bei jedem Auftreten gleich oder verschieden aus C(R¹)₂, C=O oder O ausgewählt ist, wobei R¹ aus C1-C12-Alkyl ausgewählt ist;
- L aus einer Gruppierung der Formel ------(Z)ₙ-(Ac)ₘ-Ar gemäß nachstehender Definition oder aus einer Gruppierung der Formel (19) 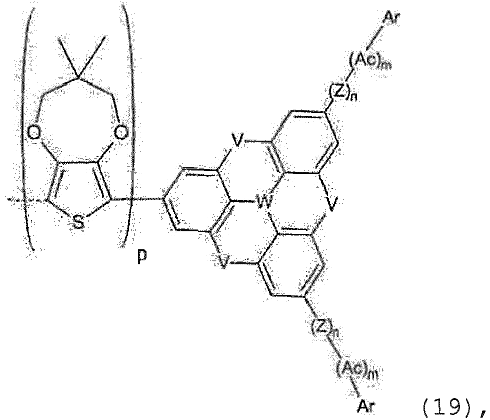 wobei p für 0, 1 oder 2 steht, und einer Gruppierung der Formel ------(Z)ₙ-(Ac)ₘ-Ar gemäß nachstehender Definition ausgewählt ist;
- Z für ein heteroaromatisches Ringsystem steht, das bei jedem Auftreten gleich oder verschieden aus einer C4-C20-Heteroaryl- und C4-C20-Heteroaryloxygruppe ausgewählt ist, wobei die Heteroatome aus O, S, Se, Si ausgewählt sind und wobei die Heteroaryl- und Heteroaryloxygruppen durch C1-C20-Alkyl, C1-C20-Heteroalkyl, C2-C20-Alkenyl oder C2-C20-Alkinyl substituiert sind, wobei das Alkyl, Alkenyl und Alkinyl dann, wenn sie 3 oder mehr Kohlenstoffatome aufweisen, linear, verzweigt oder cyclisch sein können, und n für 0, 1, 2 oder 3 steht;
- Ac für eine Akzeptorgruppe steht, die bei jedem Auftreten gleich oder verschieden aus einer Gruppierung gemäß einer der Formeln (1) bis (18) ausgewählt ist, 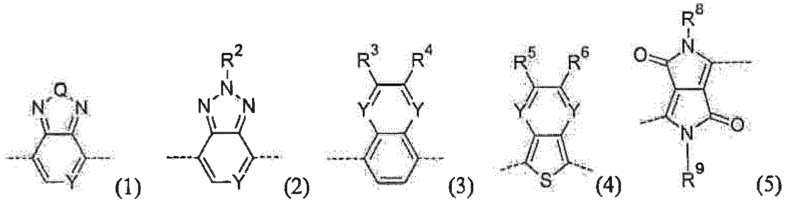 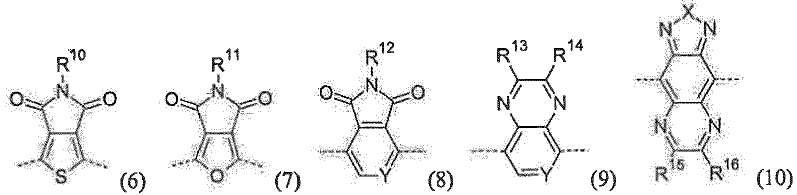 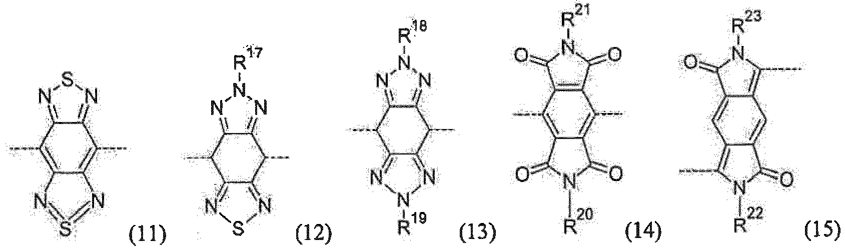 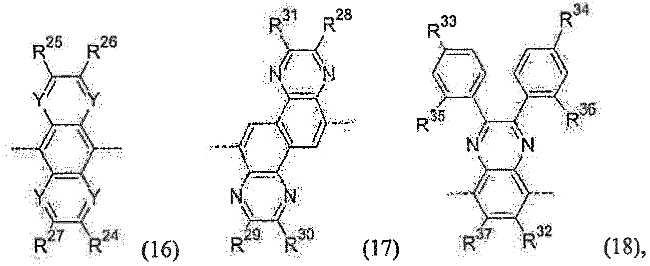
- wobei
- R² bis R³⁷ für Substituenten stehen, die unabhängig aus H, einer Ethergruppe, C1-C16-Alkyl, einer C1-C16-Alkoxygruppe, C1-C16-Thioalkyl, C1-C16-Alkoxyalkyl, C4-C16-Aryl, C1-C16-Arylalkyl oder C4-C16-Heteroaryl, C4-C16-Heteroarylalkyl ausgewählt sind, wobei die Heteroatome aus O, S oder N ausgewählt sind,
- Q aus S, O, Se, Si oder N ausgewählt sind und dann, wenn Q für N steht, das Stickstoffatom durch einen wie für R² bis R³⁷ definierten Substituenten substituiert ist,
- Y bei jedem Auftreten gleich oder verschieden aus N oder C, das unsubstituiert oder durch Halogen, das aus F, Cl, I und BR ausgewählt ist, substituiert ist, und
- m für 0 oder 1 steht;
- -Ar für ein System von aufeinanderfolgenden gebundenen heteroaromatischen Ringen mit 2 bis 10 heteroaromatischen Ringen steht, wobei die heteroaromatischen Ringe bei jedem Auftreten gleich aus einer C4-C20-Heteroaryl- und C4-C20-Heteroaryloxygruppe ausgewählt sind, wobei die Heteroatome aus O, S, Se, Si ausgewählt sind und wobei die Heteroaryl- und Heteroaryloxygruppen unsubstituiert oder durch -COOH, C4-C16-Cyanoalkenylcarbonsäure, C1-C20-Alkyl, C1-C20-Heteroalkyl, C2-C20-Alkenyl oder C2-C20-Alkinyl substituiert sind, wobei das Alkyl, Alkenyl und Alkinyl dann, wenn sie 3 oder mehr Kohlenstoffatome aufweisen, linear, verzweigt oder cyclisch sein können.

2. Verbindung nach Anspruch 1, wobei L für eine Gruppierung der Formel ------(Z)ₙ-(Ac)ₘ-Ar steht, und bei der es sich um eine Verbindung der Formel (II) handelt:

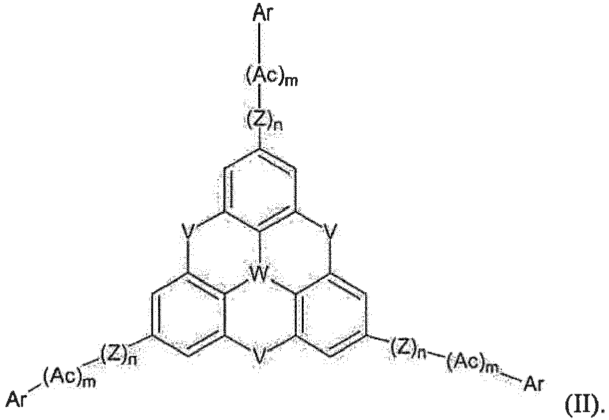

3. Verbindung nach einem der vorhergehenden Ansprüche, wobei Ac aus einer Gruppierung gemäß einer der Formeln (1) bis (4) und (10) bis (13) ausgewählt ist.

4. Verbindung nach einem der vorhergehenden Ansprüche, wobei Ac aus einer Gruppierung gemäß einer der Formeln (1), (10) und (11) ausgewählt ist.

5. Verbindung nach einem der vorhergehenden Ansprüche, wobei Q einer der Formeln (1) bis (18) aus S, O oder Se ausgewählt ist.

6. Verbindung nach einem der vorhergehenden Ansprüche, wobei Y einer der Formeln (1) bis (18) für C steht.

7. Verfahren nach einem der vorhergehenden Ansprüche, wobei Z aus einer Gruppierung gemäß einer der Formeln (20) bis (27) ausgewählt ist: 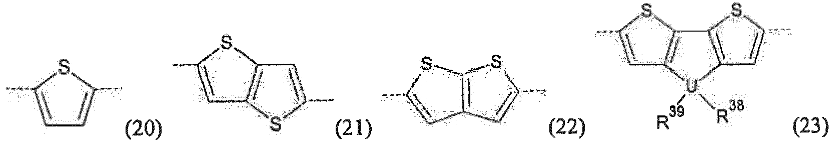 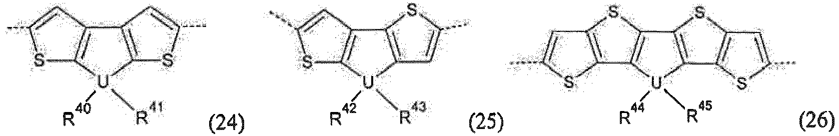 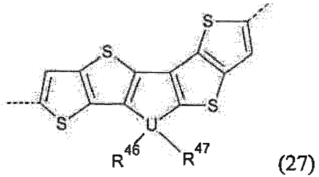
- wobei
- R³⁹ bis R⁴⁷ für Substituenten stehen, die unabhängig aus H, C1-C16-Alkyl, einer C1-C16-Alkoxygruppe, C1-C16-Thioalkyl, C1-C16-Alkoxyalkyl ausgewählt sind und dann, wenn sie 3 oder mehr Kohlenstoffatome aufweisen, linear oder verzweigt sein können;
- U aus S, Si oder C ausgewählt ist.

8. Verbindung nach Anspruch 7, wobei Z aus einer Gruppierung einer der Formeln (20), (23) bis (26) ausgewählt ist.

9. Festkörper-Photovoltaikvorrichtung, umfassend eine Verbindung nach einem der Ansprüche 1 bis 8.

10. Vorrichtung nach Anspruch 9, ferner umfassend einen organisch-anorganischen Perowskit als Sensibilisator, wobei der Perowskit in Schichtform vorliegt.

11. Vorrichtung nach Anspruch 10, wobei das Material für die Schicht aus organisch-anorganischen Perowskit eine Perowskit-Struktur einer der nachstehenden Formeln (I), (II), (III), (IV), (V) und/oder (VI) umfasst:
| | |
|---|---|
| AA'MX₄ | (I) |
| AMX₃ | (II) |
| AA'N2_{/3}X₄ | (III) |
| AN_{2/3}X₃ | (IV) |
| BN_{2/3}X₄ | (V) |
| BMX₄ | (VI), |
wobei
- A und A' für organische, einwertige Kationen stehen, die unabhängig aus primären, sekundären, tertiären oder quaternären organischen Ammoniumverbindungen einschließlich N-haltiger Heteroringe und Ringsysteme ausgewählt sind, wobei A und A' unabhängig 1 bis 60 Kohlenstoffatome und 1 bis 20 Heteroatome aufweisen;
- B für ein organisches, zweiwertiges Kation steht, das aus primären, sekundären, tertiären oder quaternären organischen Ammoniumverbindungen mit 1 bis 60 Kohlenstoffatomen und 2-20 Heteroatomen und mit zwei positiv geladenen Stickstoffatomen ausgewählt ist;
- M für ein zweiwertiges Metallkation steht, das aus der Gruppe bestehend aus Cu²⁺, Ni²⁺, Co²⁺, Fe²⁺, Mn²⁺, Cr²⁺, Pd²⁺, Cd²⁺, Ge²⁺, Sn²⁺, Pb²⁺, Eu²⁺, oder Yb²⁺ ausgewählt ist;
- N aus der Gruppe bestehend aus Bi³⁺ und Sb³⁺ ausgewählt ist; und
- X unabhängig aus Cl⁻, Br⁻, I⁻, NCS⁻, CN⁻ und NCO⁻ausgewählt ist.

12. Vorrichtung nach einem der Ansprüche 10 bis 11, wobei die Sensibilisatorschicht aus organisch-anorganischen Perowskit mit einer Schicht, die die Verbindung nach einem der Ansprüche 1-8 umfasst, überzogen ist.

13. Vorrichtung nach einem der Ansprüche 9 bis 12, wobei die Vorrichtung aus einer Solarzelle, einem Heteroübergang, einer optoelektronischen Vorrichtung und einer lichtemittierenden Vorrichtung ausgewählt ist.

14. Vorrichtung nach einem der Ansprüche 9 bis 12, wobei es sich bei der Solarzelle um eine Festkörper-Solarzelle handelt.

15. Verwendung einer Verbindung nach einem der Ansprüche 1-8 als lochtransportierendes und lichtabsorbierendes Material in einer Festkörper-Photovoltaikvorrichtung.

## Revendications

1. Composé de formule (I) : 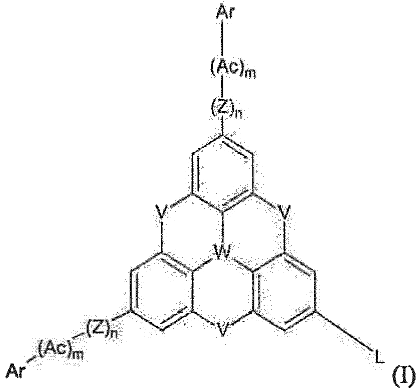 dans laquelle
- W est un atome d'azote (N) ;
- V est choisi, lors de chaque occurrence, de façon identique ou différente parmi C(R¹)₂, C=O ou O, R¹ étant choisi parmi les groupements alkyle en C1-C12 ;
- L est choisi parmi une entité de formule ------(Z)ₙ₋(Ac)ₘ-Ar comme défini ci-après ou parmi une entité de formule (19) 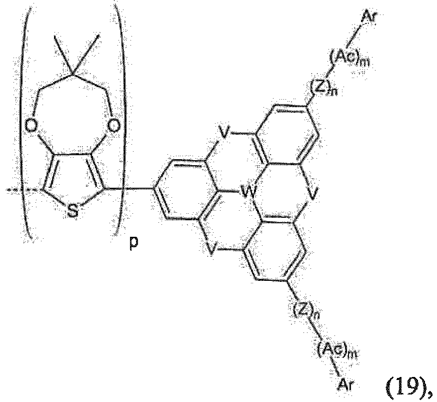 p valant 0, 1 ou 2 et l'entité de formule ------(Z)ₙ-(Ac)ₘ-Ar étant telle que définie ci-après ;
- Z représente un système cyclique hétéroaromatique, choisi lors de chaque occurrence de façon identique ou différente parmi les groupes hétéroaryle en C4-C20 et hétéroaryloxy en C4-C20, dans lesquels les hétéroatomes sont choisis parmi 0, S, Se, Si et dans lesquels lesdits groupes hétéroaryle et hétéroaryloxy sont substitués par alkyle en C1-C20, hétéroalkyle en C1-C20, alcényle en C2-C20 ou alcynyle en C2-C20, dans lesquels lesdits groupes alkyle, alcényle et alcynyle, s'ils comprennent 3 atomes de carbone ou plus, peuvent être linéaires, ramifiés ou cycliques, et n vaut 0, 1, 2 ou 3 ;
- Ac représente un groupe accepteur choisi, lors de chaque occurrence, de façon identique ou différente parmi une entité selon l'une quelconque des formules (1) à (18), 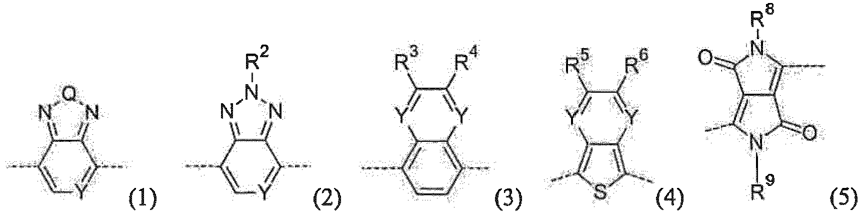 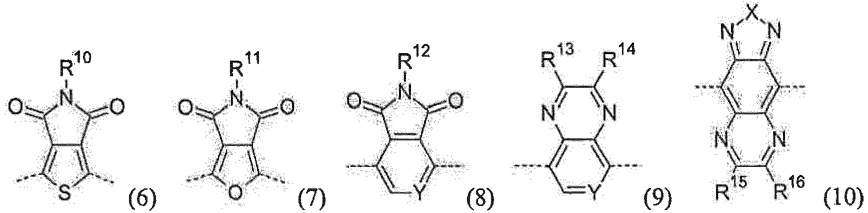 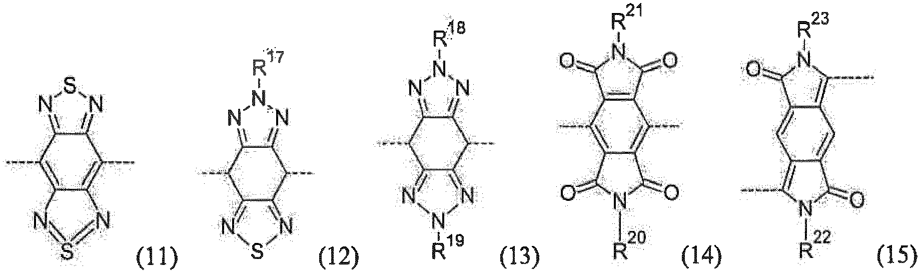 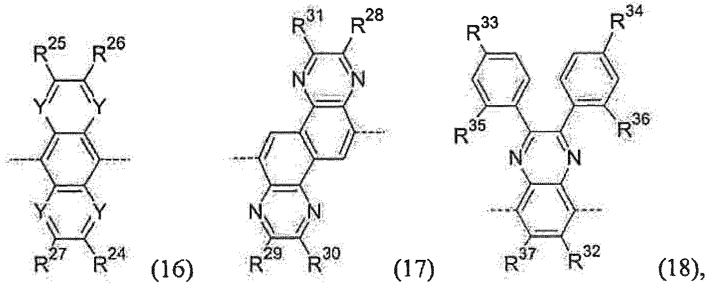
- dans lesquelles
- les radicaux R² à R³⁷ sont des substituants indépendamment choisis parmi H et les groupes éther, alkyle en C1-C16, alkoxy en C1-C16, thioalkyle en C1-C16, alkoxyalkyle en C1-C16, aryle en C4-C16, arylalkyle en C1-C16 ou hétéroaryle en C4-C16, hétéroarylalkyle en C4-C16, les hétéroatomes étant choisis parmi O, S ou N,
- Q est choisi parmi S, 0, Se, Si ou N et lorsque Q représente N, ledit atome d'azote est substitué par un substituant comme défini pour R² à R³⁷,
- Y est choisi, lors de chaque occurrence, de façon identique ou différente parmi N ou C, non substitué ou substitué par un halogène choisi parmi F, Cl, I et Br, et
- m vaut 0 ou 1 ;
- Ar représente un système de cycles hétéroaromatiques liés de façon successive comprenant entre 2 et 10 cycles hétéroaromatiques, lesdits cycles hétéroaromatiques étant, lors de chaque occurrence, choisis de façon identique parmi les groupes hétéroaryle en C4-C20 et hétéroaryloxy en C4-C20, où les hétéroatomes sont choisis parmi 0, S, Se, Si et dans lesquels lesdits groupes hétéroaryle et hétéroaryloxy sont non substitués ou substitués par - COOH, acide (cyanoalcényle en C4-C16)-carboxylique, alkyle en C1-C20, hétéroalkyle en C1-C20, alcényle en C2-C20 ou alcynyle en C2-C20, dans lesquels lesdits groupes alkyle, alcényle et alcynyle, s'ils comprennent 3 atomes de carbone ou plus, peuvent être linéaires, ramifiés ou cycliques.

2. Composé selon la revendication 1, dans lequel L ------(Z)ₙ-(Ac)ₘ-Ar représente une entité de formule et est un composé de formule (II) :

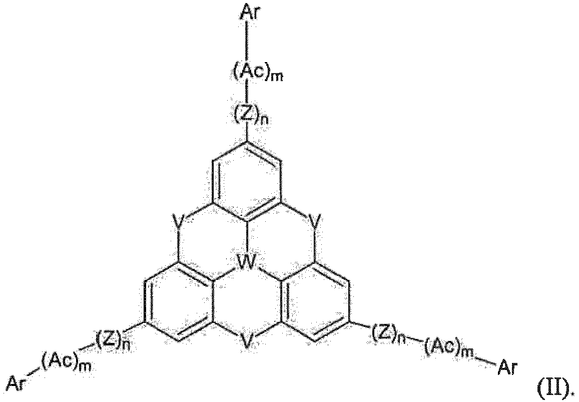

3. Composé selon l'une quelconque des revendications précédentes, dans lequel Ac est choisi parmi une entité selon l'une quelconque des formules (1) à (4) et (10) à (13).

4. Composé selon l'une quelconque des revendications précédentes, dans lequel Ac est choisi parmi une entité selon l'une quelconque des formules (1), (10) et (11).

5. Composé selon l'une quelconque des revendications précédentes, dans lequel Q dans l'une quelconque des formules (1) à (18) est choisi parmi S, 0 ou Se.

6. Composé selon l'une quelconque des revendications précédentes, dans lequel Y dans l'une quelconque des formules (1) à (18) représente C.

7. Composé selon l'une quelconque des revendications précédentes, dans lequel Z est choisi parmi une entité selon l'une quelconque des formules (20) à (27) 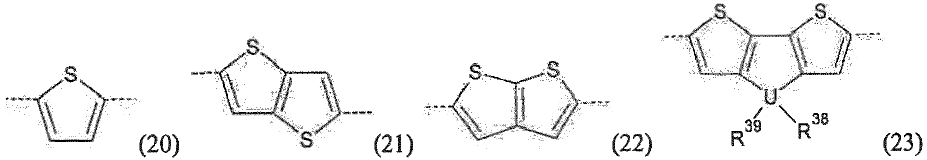 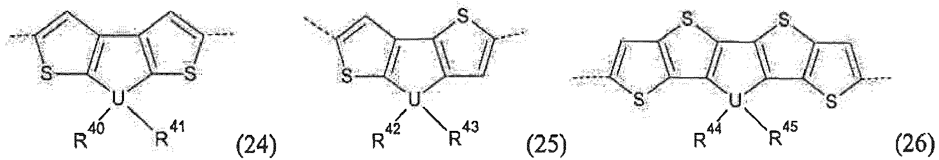 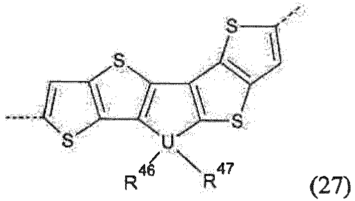
- dans lesquelles
- les radicaux R³⁹ à R⁴⁷ sont des substituants indépendamment choisis parmi H et les groupes alkyle en C1-C16, alkoxy en C1-C16, thioalkyle en C1-C16, alkoxyalkyle en C1-C16, et s'ils comprennent 3 atomes de carbone ou plus, ils peuvent être linéaires ou ramifiés ;
- U est choisi parmi S, Si ou C,

8. Composé selon la revendication 7, dans lequel Z est choisi parmi une entité selon l'une quelconque des formules (20), (23) à (26).

9. Dispositif photovoltaïque à semi-conducteurs comprenant un composé selon l'une quelconque des revendications 1 à 8.

10. Dispositif selon la revendication 9 comprenant une pérovskite organique-inorganique en tant qu'agent sensibilisant, ladite pérovskite se présentant sous la forme d'une couche.

11. Dispositif selon la revendication 10, dans lequel le matériau de la couche de pérovskite organique-inorganique comprend une structure de pérovskite selon l'une quelconque des formules (I), (II), (III), (IV), (V) et/ou (VI) ci-après :
AA'MX₄ (I)
AMX₃ (II)
AA'N_{2/3}X₄ (III)
AN_{2/3}X₃ (IV)
BN_{2/3}X₄ (V)
BMX₄ (VI)
dans lesquelles,
- A et A' sont des cations monovalents organiques indépendamment choisis parmi des composés d'ammonium organiques primaires, secondaires, tertiaires ou quaternaires, y compris des systèmes cycliques et des hétérocycles azotés, A et A' comportant indépendamment entre 1 et 60 atomes de carbone et entre 1 et 20 hétéroatomes ;
- B représente un cation organique divalent choisi parmi les composés d'ammonium organiques primaires, secondaires, tertiaires ou quaternaires, comportant entre 1 et 60 atomes de carbone et entre 1 et 20 hétéroatomes ainsi que deux atomes d'azote à charge positive ;
- M représente un cation métallique divalent choisi dans le groupe constitué par Cu²⁺, Ni²⁺, Co²⁺, Fe²⁺, Mn²⁺, Cr²⁺, Pd²⁺, Cd²⁺, Ge²⁺, Sn²⁺, Pb²⁺, Eu²⁺ ou Yb²⁺ ;
- N est choisi dans le groupe constitué par Bi³⁺ et Sb³⁺ ; et,
- X est indépendamment choisi parmi Cl⁻, Br⁻, I⁻, NCS⁻, CN- et NCO⁻.

12. Dispositif selon l'une quelconque des revendications 10 à 11, dans lequel la couche d'agent sensibilisant faite de pérovskite organique-inorganique est revêtue d'une couche comprenant le composé selon l'une quelconque des revendications 1 à 8.

13. Dispositif selon l'une quelconque des revendications 9 à 12, ledit dispositif étant choisi parmi une cellule solaire, une hétérojonction, un dispositif optoélectronique et un dispositif photoémetteur.

14. Dispositif selon l'une quelconque des revendications 9 à 12, dans lequel la cellule solaire est une cellule solaire à semi-conducteurs.

15. Utilisation d'un composé selon l'une quelconque des revendications 1 à 8 en tant que matériau transportant des trous et absorbant la lumière dans un dispositif photovoltaïque à semi-conducteurs.
